# EUROPÄISCHE PATENTANMELDUNG

(11) **EP 4 527 588 A1**
(43) Veröffentlichungstag der Anmeldung: **26.03.2025**
(21) Anmeldenummer: 24201465.2
(22) Anmeldetag: 19.09.2024
(51) Int. Cl.: B29C 49/42, B29C 49/64, B29C 49/78, B65C 3/26, B65C 9/40, A61L 2/00, B08B 9/08, B41J 3/00, B67C 3/00, B67C 3/24

(54) **VORRICHTUNG UND EIN VERFAHREN ZUM BEHANDELN VON GETRÄNKEBEHÄLTNISSEN**

(30) Priorität: 21.09.2023 DE 102023125643
(71) Anmelder: Krones AG, 93073 Neutraubling (DE)
(72) Erfinder: Fleischmann, Hans-Juergen, 93073 Neutraubling (DE); Gerstenberg, Thomas, 93073 Neutraubling (DE); Hoellriegl, Thomas, 93073 Neutraubling (DE); Kursawe, Andreas, 93073 Neutraubling (DE)
(74) Vertreter: Bittner, Bernhard

(57) **Zusammenfassung**

Vorrichtung (10, 20, 30, 40) zum Behandeln von Behältnissen (2) mit einer Transporteinrichtung (50), welche die zu behandelnden Behältnisse (2) entlang eines vorgegebenen Transportpfads (TR) transportiert und mit wenigstens einer Behandlungseinrichtung (17, 18, 26, 27, 36, 37, 38, 39, 162), welche dazu geeignet und bestimmt ist, ein Behältnis (2) in einer vorgegebenen Weise zu behandeln, wobei die Vorrichtung (1) eine Bewegungseinrichtung (14, 24, 34, 54, 64, 161) aufweist, und diese Bewegungseinrichtung wenigstens eine erste Führungskurve (14, 24, 34, 54, 64) aufweist, sowie wenigstens eine Führungsrolle (161), welche dazu geeignet und bestimmt ist, gegenüber der Führungskurve (14, 24, 34, 54, 64) abzurollen und wobei die Vorrichtung (1) weiterhin eine Erfassungseinrichtung (15, 65) aufweist, welche dazu geeignet und bestimmt ist, wenigstens einen Messwert zu erfassen, der für eine Laufeigenschaft der Führungsrolle (161) gegenüber der Führungskurve (14, 24, 34, 54, 64) charakteristisch ist, dadurch gekennzeichnet, dass die Vorrichtung (1) eine Auswerteeinrichtung (100) aufweist, welche dazu geeignet und bestimmt ist, diesen erfassten Messwert unter Berücksichtigung wenigstens eines weiteren für den Betrieb der Vorrichtung charakteristischen Betriebswertes auszuwerten.

## Beschreibung

Die vorliegende Erfindung betrifft eine Vorrichtung und ein Verfahren zum Behandeln von Getränkebehältnissen. Aus dem Stand der Technik sind diverse Vorrichtungen und Verfahren dieser Art bekannt. So sind beispielsweise Umformungseinrichtungen wie beispielsweise Streckblasmaschinen bekannt, welche Kunststoffvorformlinge zu Kunststoffflaschen umformen. Daneben sind auch Inspektionseinrichtungen bekannt, welche Behältnisse inspizieren oder auch Etikettiereinrichtungen, welche Behältnisse mit einem Etikett versehen.

Bei diesen Vorrichtungen werden üblicherweise die zu behandelnden Behältnisse transportiert und es wird an diesen Behältnissen ein Behandlungsvorgang vorgenommen wie beispielsweise ein Etikettiervorgang, ein Umformungsvorgang oder dergleichen. Zu diesem Zweck ist es üblich, dass Behandlungselemente wie etwa eine Blasdüse auf die Kunststoffvorformlinge zugestellt werden, um diese dann zu expandieren.

Im Stand der Technik sind unterschiedliche Vorgehensweisen bekannt, wie diese Bewegungen realisiert werden. So wäre es möglich, den einzelnen Behandlungselementen jeweils eigene Antriebe wie Elektromotoren zuzuordnen. Eine seit langer Zeit bewährte Vorgehensweise ist jedoch das Vorsehen von Führungskurven, an denen Führungsrollen abrollen und die Position der Führungskurve legt die Position des Behandlungselements fest. Diese Technologie ist bewährt und seit langem Standard bei derartigen Maschinen.

Derartige Kurvensysteme sind jedoch einem Verschleiß unterworfen, der mit der Zeit dazu führen kann, dass die Bewegungen nicht mehr präzise durchgeführt werden.

Aus der DE 10 2017 120 295 A1 sind eine Behälterbehandlungsanlage und ein Verfahren zur Erfassung von Laufeigenschaften einer Rolle an einer Führungskurve einer solchen Behälterbehandlungsanlage bekannt. In diesem Dokument wird beschrieben, dass einzelne Bewegungen mittels Sensoreinrichtungen erfasst werden, sodass Informationen für einen Verschleiß der Hubkurve, im Folgenden jedoch als Führungskurve bezeichnet oder auch der Rollen, im Folgenden als Führungsrollen bezeichnet ermittelt werden können.

Der Offenbarungsgehalt der DE 10 2017 120 2951 wird durch Bezugnahme vollständig auch zum Gegenstand der vorliegenden Patentanmeldung gemacht.

Aus dem internen Stand der Technik der Anmelderin ist es bekannt, derartige Maschinen mit Schwingungssensoren an Führungskursen wie etwa der Hauptkurve, einer Verriegelungskurve und einer Reckstangenkurve vorzusehen. Bei Überschreiten von bestimmten Grenzwerten werden je nach dem überschrittenen Grenzwert entweder Warnmeldungen (beispielsweise an einer Anzeigeeinrichtung) angezeigt oder wird die Produktion beendet und die Maschine stillgesetzt oder auch ein Schnellstopp der Maschine eingeleitet.

Es gibt jedoch verschiedene Ursachen, welche diese erhöhten Schwingungswerte hervorrufen können. Bei Überschreiten eines bestimmten Grenzwerts soll durch einen Schnellstopp ein Maschinenschaden verhindert werden.

Eine Problematik hierbei ist, dass beim Durchfahren der Führungskurven eine Vielzahl von Führungsrollen beispielsweise von Blasstationen zeitgleich im Eingriff sind. Durch diesen zeitgleichen Eingriff mehrerer Führungsrollen in der Kurve kann ein erhöhter Schwingungswert nicht immer sicher einer bestimmten Umformungsstation oder allgemein Behandlungsstation zugeordnet werden.

Da der Fehler nicht sicher einer bestimmten Behandlungsstation zugeordnet werden kann, ist die Suche nach der Fehlerursache aufwendiger, da mehrere Behandlungsstationen überprüft werden müssen.

Es können auch keine Rückschlüsse auf den Zustand einzelner Führungsrollen gezogen werden.

Im Stand der Technik erfolgt daher eine Reaktion bei der Überschreitung eines bestimmten Grenzwerts. Mögliche Einflüsse auf die Schwingungswerte wie beispielsweise das produzierte Kundenobjekt, die Maschinengeschwindigkeit, die Laufzeit der Maschine oder Umgebungsbedingungen werden nicht berücksichtigt.

Daneben wird auch eine Historie, beispielsweise ein Trendverlauf nicht berücksichtigt, um damit Prognosen für die verbleibende Laufzeit oder auch bis zu einem Ausfall geben zu können.

Der vorliegenden Erfindung liegt daher die Aufgabe zugrunde, eine Beurteilung oder auch Prognose von auftretenden Fehlern zu ermöglichen bzw. zu vereinfachen. Dies wird erfindungsgemäß durch die Gegenstände der unabhängigen Patentansprüche erreicht. Vorteilhafte Ausführungsformen und Weiterbildungen sind Gegenstand der Unteransprüche.

Eine erfindungsgemäße Vorrichtung zum Behandeln von Behältnissen weist eine Transporteinrichtung auf, welche die zu behandelnden Behältnisse entlang eines vorgegebenen Transportpfades transportiert und wenigstens eine Behandlungseinrichtung, welche dazu geeignet und bestimmt ist, wenigstens ein Behältnis und insbesondere wenigstens ein von der Transporteinrichtung transportiertes Behältnis in einer vorgegebenen Weise zu behandeln, wobei die Vorrichtung eine Bewegungseinrichtung aufweist, (welche bevorzugt dazu geeignet und bestimmt ist, eine Relativbewegung und insbesondere zwischen dem zu behandelnden Behältnis und der Behandlungseinrichtung und/oder wenigstens einem Element dieser Behandlungseinrichtung in einer Bewegungsrichtung zu bewirken).

Es wird darauf hingewiesen, dass bei manchen Maschinengattungen bevorzugt die Transproteinrichtung auch eine Behandlungseinrichtung sein kann. So wirken etwa die Umformungsstationen einerseits als Behandlungseinrichtungen, welche Kunststoffvorformlinge zu Kunststoffbehältnissen umformen, andererseits jedoch auch Transporteinrichtungen, weil sie die Kunststoffvorformlinge entlang eines vorgegebenen Transportpfads transportieren (insbesondere während der Behandlung).

Dabei weist diese Bewegungseinrichtung wenigstens eine erste Führungskurve auf, sowie wenigstens eine Führungsrolle, welche dazu geeignet und bestimmt ist, gegenüber der Führungskurve abzurollen (bevorzugt rollt diese Führungsrolle gegenüber wenigstens einer Abrollfläche der Führungskurve ab). Weiterhin weist die Vorrichtung wenigstens eine Erfassungseinrichtung auf, welche dazu geeignet und bestimmt ist, wenigstens einen Messwert zu erfassen, der für eine Laufeigenschaft der Führungsrolle gegenüber der Führungskurve charakteristisch ist.

Unter einer Laufeigenschaft der Führungsrolle gegenüber der Führungskurve wird insbesondere jegliche Charakteristik dieses Abrollens bezeichnet, wie etwa etwaige Unwuchten beim Abrollen, durch eine beschädigte Oberfläche der Führungsrolle verursachte Schwingungen, durch eine beschädigte Oberfläche der Führungskurve verursachte Schwingungen und dergleichen.

Erfindungsgemäß weist die Vorrichtung eine Auswerteeinrichtung auf, welche dazu geeignet und bestimmt ist, diesen erfassten Messwert unter Berücksichtigung wenigstens eines weiteren für den Betrieb der Vorrichtung und/oder einer Behältnisbehandlungsanlage charakteristischen Betriebswertes auszuwerten, wobei die Vorrichtung ein Bestandteil dieser Behandlungsanlage ist.

Es wird daher vorgeschlagen, dass die Messwerte nicht nur als solche erfasst werden und abgespeichert werden und aus diesen direkt eine Information abgeleitet wird, sondern dass eine Auswertung auch unter Berücksichtigung des genannten für den Betrieb der Vorrichtung charakteristischen (Betriebs)wertes erfolgt. Bei diesem charakteristischen (Betriebs)wert kann es sich, wie unten genauer dargestellt beispielsweise um eine Produktionsgeschwindigkeit handeln, aber auch um Umgebungswerte wie eine Temperatur und dergleichen.

Daneben kann es sich bei diesem charakteristischen Wert auch um einen Wert handeln, der für den Betrieb der Vorrichtung selbst charakteristisch ist, wie etwa eine Reckstangenposition einer bestimmten Umformungsstation (insbesondere einer Umformungsstation, der eine überwachte Führungsrolle zugeordnet ist oder dergleichen. Daneben kann es sich bei dem Betriebswert auch um einen Betriebswert handeln, der für eine weitere Vorrichtung (der gleichen Behältnisbehandlungsanlage) charakteristisch ist. Auch kann es sich bei dem Betriebswert um einen Wert handeln, der für das von der Vorrichtung behandelte Behältnis charakteristisch ist.

In einer bevorzugten Ausführungsform wird mindestens eine Rolle mit einer vorgegeben Kraft gegen eine Führungskurve gedrückt und/oder gedrängt (dies kann beispielsweise bei Verriegelungs- und Blasdüsenkurve der Fall sein oder z.B. bei einer Etikettierkurve). Bevorzugt tritt hier ein aktives Drücken der Rolle gegen die Kurve auf (beispielsweise unter Verwendung einer Feder).

In einer weiteren Ausführungsform werden mindestens zwei Rollen in einer Kurve und insbesondere einer Nutkurve zwangsgeführt (bei den Maschinen der Anmelderin kann es sich hier etwa um eine Hauptkurve oder eine Kurve zur Bewegung von Schwenkarmen in einem Teilungsverzugsstern handeln. In diesem Fall kommt es insbesondere aufgrund eines Bewegungsablauf zu einer Führung und/oder einem Drängen und/oder einer Anpresskraft.

Der Erfindung liegt daher die Idee zugrunde, dass nicht nur die Bewegung der Führungsrollen selbst überwacht wird, sondern diese Bewegung auch im Kontext anderer Größen überwacht wird. Damit können genauere Aussagen dahingehen getroffen werden, ob ein bestimmtes Signal der Erfassungseinrichtung auf einem Fehler einer bestimmten Führungsrolle beruht oder andere Ursachen hat, welche durch den Betrieb der Vorrichtung oder einer übergeordnete Behältnisbehandlungseinrichtung verursacht sind.

Bevorzugt ist die Auswerteeinrichtung dazu geeignet und bestimmt, einen Ergebniswert auszugeben, insbesondere einen Ergebniswert, aus dem ein Benutzer ableiten kann, wie er verfahren soll. So kann beispielsweise ein Ergebniswert indizieren, dass eine Führungsrolle einer bestimmten Umformungsstation in Kürze ausfallen wird. Auch kann ein Ergebniswert indizieren, dass eine Führungsrolle, welche einer bestimmten Halteeinrichtung zum Halten von Behältnissen zugeordnet ist, in Kürze ausfallen wird. Auch kann der Ergebniswert einer Anlagensteuerung zugeführt werden, welche dann entsprechende Steuerungen der Behältnisbehandlungsanlage initiiert wie etwa einen langsameren Betrieb oder auch einen Maschinenstopp.

Besonders bevorzugt ist wenigstens eine Halteeinrichtung und ist bevorzugt eine Vielzahl von Halteeinrichtungen zum Halten einzelner Behältnisse vorgesehen. Bevorzugt weist die Transporteinrichtung eine Vielzahl solcher Halteeinrichtungen auf.

Bevorzugt weist die Transporteinrichtung einen bewegbaren und insbesondere drehbaren Träger auf, an welchem diese Halteeinrichtungen angeordnet sind.

Bei einer bevorzugten Ausführungsform ist jeder Halteeinrichtung eine Behandlungseinrichtung zugeordnet. Umgekehrt ist bevorzugt jeder Behandlungseinrichtung mindestens eine und bevorzugt genau eine Halteeinrichtung zugeordnet. Bei einer weiteren bevorzugten Ausführungsform ist jeder Behandlungseinrichtung und/oder jeder Halteeinrichtung wenigstens eine Führungsrolle zugeordnet. Es wäre jedoch auch möglich, dass einer bestimmten Behandlungseinrichtung mehrere Führungsrollen zugeordnet sind (etwa um unterschiedliche Bewegungen durchzuführen wie etwa eine Schließbewegung von Blasformen oder eine Reckstangenbewegung).

Die besagte Kurvenrolle ist dabei bevorzugt entweder mit der Behandlungseinrichtung oder mit der dieser Behandlungseinrichtung zugeordneten Halteeinrichtung verbunden, sodass die Behandlungseinrichtung oder die Halteeinrichtung einer Bewegung der Kurvenrolle (in einer von der Transportrichtung der Behältnisse abweichenden Richtung) folgt. Es wäre jedoch auch möglich, dass sowohl die Halteeinrichtungen als auch die Behandlungseinrichtungen mit einer Kurvenrolle gekoppelt sind.

Daher bewirkt die Führungskurve beispielsweise eine Bewegung der Kurvenrolle in einer zu dem Transportpfad (der Behältnisse) senkrecht stehenden Richtung, und die mit der Führungsrolle gekoppelte Behandlungseinrichtung oder die Halteeinrichtung für die Behältnisse folgt dieser Bewegung.

Bei einer bevorzugten Ausführungsform ist die Bewegungsrichtung senkrecht zu dem Transportpfad der Behältnisse. Bevorzugt ist die Führungskurve stationär angeordnet.

Bei einer weiteren vorteilhaften Ausführungsform weist die Vorrichtung eine zweite Führungskurve auf, die wenigstens eine weitere Bewegung der Behandlungseinrichtung oder eines Bestandteils dieser Behandlungseinrichtung oder einer anderen Behandlungseinrichtung steuert bzw. bewirkt. Bevorzugt weist auch diese zweite Führungskurve wenigstens eine Erfassungseinrichtung auf, welche dazu geeignet und bestimmt ist, wenigstens einen zweiten Messwert zu erfassen, der für eine Laufeigenschaft der Rolle gegenüber der Führungskurve charakteristisch ist.

Bevorzugt ist in diesem Fall die Auswerteeinrichtung dazu geeignet und bestimmt, die Messwerte beider Erfassungseinrichtungen zu berücksichtigen, wobei in diesem Fall der Messwert der zweiten Erfassungseinrichtung der weitere für den Betrieb der Vorrichtung charakteristische (Betriebs)wert ist.

So kann beispielsweise im Falle einer Blasformmaschine ein Öffnungsmechanismus, der die Blasformen öffnet und schließt mittels einer Führungskurve angetrieben werden und andererseits auch eine Bewegung einer Reckstange oder auch einer Blasdüse, welche auf den zu expandierenden Kunststoffvorformlinge zugestellt wird, von einer (weiteren) Führungskurve bewegt werden.

Die vorliegende Erfindung wird im Folgenden unter Bezugnahme auf eine Umformungseinrichtung zum Umformen von Kunststoffvorformlingen zu Kunststoffbehältnissen sowie auch unter Bezugnahme auf eine Etikettiermaschine beschrieben. Es wird jedoch darauf hingewiesen, dass die Erfindung auch auf andere Maschinengattungen anwendbar ist, wie etwa Füllmaschinen, Sterilisationsmaschinen oder Inspektionsmaschinen.

Im Falle einer Maschine sind aus dem internen Stand der Technik der Anmeldering bereits Erfassungsvorrichtungen bekannt (dabei ist ein spezieller Ausschnitt in der Führungskurve vorgesehen sowie ein Sensor). Diese werden dabei im Zusammenhang mit einer Führungskurve verwendet, insbesondere um einzelne Rollen überwachen zu können.

Es wurde bereits versucht, dieses System in einem ersten Schritt in der Hauptkurve (einer Umformungseinrichtung) einzusetzen. Daneben soll jedoch auch eine Möglichkeit geschaffen werden, mit diesem System einen Defekt an einem Lager einer (bestimmten) Führungsrolle zu erfassen.

In einem zweiten Schritt wird versucht, dieses System auch für eine Blasdüsenkurve und weitere Kurven wie beispielsweise bei einem Teilungsverzugsstern zu verwenden.

Bevorzugt wird dieses beschriebene System für sämtliche Kurvenanwendungen dementsprechend angepasst, ausgelegt und integriert.

Bevorzugt werden die Messwerte dieser Erfassungseinrichtungen, beispielsweise Schwingungssensoren, insbesondere gemeinsam mit anderen Maschinenparametern wie etwa der Maschinengeschwindigkeit oder der Maschinenleistung an ein übergeordnetes Datenerfassungssystem übergeben und dort bevorzugt verarbeitet und gespeichert.

Bei dem wenigstens einen Betriebswert kann es sich um einen Maschinenparameter oder Prozessparameter (z.B. einen Parameter des Ofens oder der Blasmaschine) handeln, welcher ausgewählt ist aus einer Gruppe von Maschinenparametern, welche eine Maschinengeschwindigkeit, eine Maschinenleistung, einen Maschinenzustand (beispielsweise ein Baujahr und/oder Informationen zu einer Nachrüstung und/oder einer Maschinenzustandsüberprüfung), einen Maschinentyp (etwa Angabe zu einer Maschinengeneration), eine (aktuelle) Zeitangabe, eine für ein zu behandelndes bzw. behandeltes Behältnis charakteristische Behältnisgröße oder Preformgröße, und dergleichen sowie Kombinationen hiervon umfasst.

Bei der Behältnisgröße kann es sich um eine für eine Behältnisart (fertig ausgeformtes Behältnis oder Vorformling) und/oder einen Behältnistyp und/oder ein Behältnismaterial und/oder eine Zusammensetzung des Behältnismaterials (etwa Recyclingmaterial) und/oder eine (geometrische) Dimension des Behältnisses charakteristische Größe handeln.

Bei dem wenigstens einen Betriebswert kann es sich (zusätzlich oder alternativ) um eine Umgebungsgröße handeln, welche für eine Umgebung, in welcher die Vorrichtung aufgestellt ist, charakteristisch ist. Die Umgebungsgröße kann für eine Luftfeuchtigkeit, eine klimatische Bedingung bzw. ein Klima, eine Innen- und/oder Außentemperatur (einer Halle, in welcher die Vorrichtung aufgestellt ist), ein Luftdruck, eine für die Positionierung der Vorrichtung charakteristische (geometrische) Größe, wie etwa für einen Untergrund (Steigung, Beschaffenheit) der Maschinenhalle charakteristische Größe, und dergleichen sowie Kombinationen hierfür charakteristisch sein.

Dabei ist es möglich, dass eine Vorverarbeitung der Messwerte dieser Schwingungssensoren beispielsweise in einer Steuerung erfolgt.

Bevorzugt ist die Vorrichtung derart ausgelegt, dass eine Datenverarbeitung auch bereits in einer Maschinensteuerung erfolgt oder dass Teile hiervon oder nur einzelne Ergebnisse an ein Datenerfassungssystem weitergeleitet werden.

Daneben ist es möglich, dass die vollständige Verarbeitung in einem übergeordneten System (beispielsweise einer Produktionslinie, im Internet oder einer cloud) stattfindet, welches bevorzugt alle Daten von einer Vielzahl von Maschinen der Linie oder Produktion dementsprechend verarbeitet und analysiert.

Mit der genannten Erfassungseinrichtung, beispielsweise einem verbauten Schwingungssensor in Verbindung mit einem speziellen Ausschnitt (der Führungskurve) können bevorzugt erhöhte Schwingungen, Beschädigungen am Laufring einer Rolle und Schäden an einem Lager erkannt und bevorzugt auch einer bestimmten Behandlungsstation beispielsweise einer Umformungsstation zugeordnet werden.

Die Fehlersuche wird durch die Zuordnung zu einer bestimmten Umformungsstationen wesentlich erleichtert. Je nach einer Ausführung kann auch ein Verschleiß an einer bestimmten Führungsrolle erkannt werden.

Bei einer bevorzugten Ausführungsform weist die Vorrichtung eine Zuordnungseinrichtung auf, welche einem Messwert eine für den Betrieb der Vorrichtung charakteristischen Betriebswert zuordnet und insbesondere einen Betriebswert, der für einen Zeitraum oder Zeitpunkt charakteristisch ist, zu welchen der Messwert aufgenommen wurde und/oder zu welchen der besagte Messwert auftrat.

Durch diese Zuordnung wird eine Möglichkeit geschaffen, bestimmte Messwerte in einem Maschinenkontext sehen, beispielsweise in Verbindung mit einer momentanen Betriebsgeschwindigkeit, aber auch in Verbindung mit Umgebungsparametern. Auf Diese Weise kann ein deutlich genaueres Bild von auftretenden Messwerten gezeichnet werden.

Bei einer weiteren bevorzugten Ausführungsform weist die Vorrichtung eine Zeitgebereinrichtung auf, welche dazu geeignet und bestimmt ist, einer Messung eines Messwerts und/oder dem Messwert selbst einen Zeitraum und/oder einem Zeitpunkt, in oder zu welchem dieser Messwert gemessen wurde, zuzuordnen.

Bei einer weiteren vorteilhaften Ausführungsform weist die Vorrichtung eine Speicherungseinrichtung auf, welche zum Speichern einer Vielzahl von Messwerten geeignet und bestimmt ist, wobei bevorzugt diese Messwerte mit einer Zuordnung zu Betriebswerten abspeicherbar sind. Auf diese Weise kann eine hohe Vielzahl von Daten aufgenommen werden, wobei jeweils auch die jeweiligen Kontexte der Messwerte wie beispielsweise Betriebszustände (und bevorzugt auch Zeitpunkte zu denen sie aufgetreten sind) gespeichert werden.

Daneben können einzelnen Messwerten auch weitere Messwerte beispielsweise der gleichen Behandlungsstation zugeordnet werden. So kann beispielsweise einem bestimmten Messwert eines Schwingungssensors ein Wert einer Reckstangen Position oder ein (momentaner) Öffnungs- oder Schließzustand einer Umformungsstationen zugeordnet werden.

Bei einer weiteren vorteilhaften Ausführungsform weist die Vorrichtung eine Vielzahl von Behandlungseinrichtungen und insbesondere eine Vielzahl von gleichartigen Behandlungseinrichtungen auf, welche dazu geeignet und bestimmt sind, Behältnisse und insbesondere von der Transporteinrichtung transportierte Behältnisse in einer vorgegebenen Weise zu behandeln.

Bevorzugt ist dabei jeder dieser Behandlungseinrichtungen wenigstens eine Kurvenrolle zugeordnet. So kann es sich beispielsweise bei diesen Behandlungseinrichtungen um Umformungsstationen handeln, welche Kunststoffvorformlinge zu Kunststoffbehältnissen expandieren. Bevorzugt sind diese Behandlungseinrichtungen an einem gemeinsamen Träger angeordnet und insbesondere an einem gemeinsamen beweglichen und insbesondere drehbaren Träger.

Bevorzugt weisen diese Umformungsstationen jeweils Blasformen und/oder Blasformträger auf, die zum Öffnen und Schließen der Blasformen schwenkbar sind. Dabei wird bevorzugt dieser Schwenkvorgang durch eine Führungskurve und eine gegenüber dieser Führungskurve abrollende Führungsrolle bewirkt.

Daneben können die Umformungsstationen jeweils Verriegelungsmechanismen aufweisen, welche in einem geschlossenen Zustand der Blasformen die Seitenteile der Blasformen miteinander verriegeln.

Bevorzugt weisen diese Umformungsstationen jeweils stangenartige Körper, insbesondere sogenannte Reckstangen auf, welche in das Innere der Kunststoffvorformlinge einführbar sind, um diese in deren Längsrichtung zu denen. Auch die Bewegung dieser Reckstangen kann dabei durch eine Führungskurve und wenigstens eine der betreffenden Reckstange zugeordnete Kurvenrolle bewirkt werden.

Bevorzugt weisen die Umformungsstationen jeweils Beaufschlagungseinrichtungen auf, welche an die Kunststoffvorformlinge angelegt werden können, um diese mit einem flüssigen Medium und insbesondere mit Druckluft zu beaufschlagen. Auch diese Bewegung der Beaufschlagungseinrichtungen kann dabei von einer Führungskurve und einer dieser Führungskurve zugeordneten Führungsrolle bewirkt werden.

Besonders bevorzugt sind diese Behandlungseinrichtungen an einem gemeinsamen insbesondere beweglichen und insbesondere drehbaren Träger angeordnet, wobei vorzugsweise an diesem Träger auch die Halteeinrichtungen zum Halten der zu behandelnden Behältnisse angeordnet sind. Bevorzugt ist jeder Behandlungseinrichtung genau eine Halteeinrichtung zugeordnet. Diese Halteeinrichtung kann dabei als Greifklammer ausgebildet sein, es wäre jedoch auch denkbar, dass andere Elemente wie etwa Blasformen auch als Halteeinrichtungen dienen.

Bei einer bevorzugten Ausführungsform ist jeder der Halteeinrichtungen zum Halten der Kunststoffbehältnisse (oder Kunststoffvorformlinge) oder allgemein jedem der Behältnisse eine Führungsrolle zugeordnet, welche wenigstens eine zu dem Transportpfad senkrecht verlaufende Bewegung ausführt.

Die Behandlungseinrichtungen und/oder die Halteeinrichtungen sind bevorzugt wie erwähnt an einem gemeinsamen bewegbaren und besondere drehbaren Träger angeordnet.

Bevorzugt sind aufgezeichnete und/oder gespeicherte Messwerte auch für ein Überwachungssystem verwendbar und/oder zur Ausgabe von Meldungen an eine Anzeigeeinrichtung beispielsweise ein Handy, Tablet o.ä. Mit diesen Meldungen können bestimmte Personenkreise wie beispielsweise das Bedienungspersonal eine Instandhaltung oder dergleichen durchführen oder darüber informiert werden.

Die aktuellen Messwerte beispielsweise einer Schwingungsmessung können in Relation zu anderen die Schwingungswerte beeinflussenden Werten gesetzt werden wie beispielsweise dem produzierten Kundenobjekt, den verwendeten Kunststoffvorformlingen oder zu sonstigen Behälterparametern. Daneben können auch Maschinenparameter, Heizparameter, Produktionsparameter, gegebenenfalls auch die Laufzeit der betreffenden Maschine oder Umgebungsbedingungen mitverwendet oder gesetzt werden und damit kann bevorzugt eine Auswertung mit dynamischen und möglicherweise auch stationsspezifischen (Grenz)werten erfolgen.

Bei einer weiteren bevorzugten Ausführungsform ist auch eine Historie der Maschine und/oder Vorrichtung mit berücksichtigbar. Auf diese Weise kann ein sich abzeichnender Trend frühzeitig erkannt werden und darauf basierend eine Prognose zur verbleibenden Laufzeit bis zu einem kritischen Maschinenzustand abgegeben werden (predictive maintenance).

Bei einer bevorzugten Ausführungsform ist ein Algorithmus vorgesehen, der basierend auf den Daten mehrerer Maschinen bzw. Vorrichtungen trainiert werden kann, um im Betrieb stetig selbst dazu zu lernen. Insbesondere wird eine künstliche Intelligenz (KI) eingesetzt.

Bevorzugt werden nun von einer möglichst großen Zahl an Behältnissen und einer breiten Varianz an Produktionsdaten und/oder Umgebungsdaten und/oder Messwerten (insbesondere der für die physikalischen Eigenschaften der Tragringe charakteristischen Messwerte) alle verfügbaren Daten zusammengebracht und/oder einander zugeordnet und bevorzugt daraus ein Modell gebildet.

Zur Modellbildung können verschiedene Möglichkeiten in Betracht gezogen werden. So können beispielsweise klassische Korrelationsanalysen oder Dimensionsanalysen vorgenommen werden. Auch können mit Hilfe mathematischer Fitfunktionen Zusammenhänge modelliert werden.

Es kann weiterhin, insbesondere unter Hinzuziehen eines Experten, ein Modell erzeugt werden. Ebenfalls denkbar sind verschiedene Methoden der Kl, wie ein Neuronales Netzwerk ein Reinforcement Learning oder eine Physical Based Kl welche ein Modell erzeugen.

Bevorzugt erfolgt die Ermittlung des wenigstens einen Ergebniswertes, bevorzugt von Betriebsparametern und/oder Umgebungsparametern, mit einem, bevorzugt trainierbaren, Modell maschinellen Lernens, welches bevorzugt auf einem (künstlichen) neuronalen Netzwerk und insbesondere auf wenigstens einem, und insbesondere genau einem, (künstlichen) neuronalen Netzwerk basiert.

Die Daten, die in das Modell (als Eingangsgrößen) einfließen, können während der Standard Produktion erzeugt werden oder in speziellen Produktionsläufen, in denen gezielt bestimmte Parameter variiert werden.

Es ist auch denkbar, beide Verfahren zu kombinieren und in einem "Teachinglauf" ein Basisset an Daten für ein Basismodell zu erzeugen und dann in laufender Produktion nach und nach Daten in das System einzuspeisen und gegebenfalls das Modell nachzuschärfen.

Bevorzugt ist das neuronale Netzwerk als tiefes neuronales Netzwerk (Deep Neural Network, DNN), bei dem die parametrierbare Verarbeitungskette eine Mehrzahl von Verarbeitungsschichten aufweist, und/oder ein sogenanntes Convolutional Neural Network (CNN) (dt. Faltungsnetzwerk) und/oder ein rekurrentes Neuronales Netzwerk (RNN, engl. Recurrent Neural Network) ausgebildet.

Bevorzugt werden einem bzw. dem Modell bzw. dem (künstlichen) neuronalen Netzwerk die (zu verarbeitenden bzw. bevorzugt die von der Auswerteeinrichtung auszuwertenden) Daten, insbesondere den der Erfassungseinrichtung erfassten Messwert (bevorzugt die von den Erfassungseinrichtungen erfassten Messwerte), etwa die Daten eines Schwingungssensors (oder hiervon abgeleitete Daten) und/oder die Daten der Behandlungseinrichtung und/oder des wenigstens einen Betriebswerts (bevorzugt einer Vielzahl von Betriebswerten) als Eingangsgrößen zugeführt. Bevorzugt bildet das Modell bzw. das künstliche neuronale Netzwerk die Eingangsgrößen in Abhängigkeit einer parametrierbaren Verarbeitungskette auf Ausgangsgrößen ab, wobei als Ausgangsgröße bevorzugt der Ergebniswert und bevorzugt als Ausgangsgrößen eine Vielzahl von Ergebniswerten gewählt sind.

Bevorzugt wertet die, bevorzugt Prozessor-basierte, Auswerteeinrichtung den wenigstens einen Messwert bzw. die Vielzahl an Messwerte und den wenigstens einen Betriebswert bzw. die Vielzahl an Betriebswerten mittels des Modells aus.

Denkbar ist auch, dass dem Modell nicht nur die Eingangsgrößen bezogen auf genau eine Vorrichtung zugeführt werden, sondern die Eingangsgrößen bezogen auf wenigstens zwei und bevorzugt einer Vielzahl von Vorrichtungen. So könnte für jede Vorrichtung ein Datensatz umfassend wenigstens ein (von einer Erfassungseinrichtung erfasster) Messwert und wenigstens ein Betriebswert (bevorzugt eine Vielzahl von Betriebswerten) als Eingangsgrö-ßen zugeführt werden.

Die dem Modell als Eingangsgrößen zuzuführenden Daten können dabei (wenigstens teilweise und bevorzugt alle) von einer externen Speichereichrichtung abgerufen und/oder übermittelt werden. Bei der externen Speichereinrichtung kann es sich um eine in Bezug auf die Vorrichtung externe Speichereinrichtung handeln, welche insbesondere beabstandet von dem Betriebsgelände der Vorrichtung angeordnet ist. Auf der externen Speichereinrichtung können daher vorteilhaft die Daten mehrerer Vorrichtungen (insbesondere unabhängig von dem jeweiligen Betreiber der Vorrichtung) gesammelt und (etwa von dem externen Server) ausgewertet werden.

Bevorzugt werden dem Modell eine Vielzahl von Eingangsgrößen zugeführt, welche alle einem vorgegebenen und/oder vorgebbaren Zeitraum (in welchem diese erhoben bzw. ermittelt wurden) zugeordnet und/oder zuordenbar sind. Bevorzugt umfasst diese Vielzahl von Eingangsgrößen (desselben Erhebungszeitraums) mindestens 10, bevorzugt mindestens 50, bevorzugt mindestens 100 verschiedene Eingangsgrößen.

Dabei handelt es sich bevorzugt bei der Vielzahl von Vorrichtungen um Vorrichtungen zum Behandeln von Behältnissen mit demselben (grundsätzlichen) Behandlungsziel, also beispielsweise der Umformung von Kunststoffvorformlingen zu Kunststoffbehältnissen. Denkbar ist auch, dass mehrere oder alle der Vielzahl von Vorrichtungen baugleiche Vorrichtungen sind. Denkbar ist auch, dass es sich bei mehreren oder allen der Vielzahl von Vorrichtungen um bauähnliche Vorrichtungen oder Vorrichtungen verschiedener Generation handelt. Denkbar ist auch, dass es sich bei der Vielzahl von Vorrichtungen um Vorrichtungen desselben Herstellers handelt.

Bevorzugt sind mehrere oder alle der Vielzahl von Vorrichtungen nicht auf demselben Betriebsgelände und/oder nicht in derselben Maschinenhalle angeordnet.

Bevorzugt umfasst die Vielzahl von Vorrichtungen mindestens zwei, bevorzugt mindestens 5, bevorzugt mindestens 10 und besonders bevorzugt mindestens 100 voneinander (paarweise) verschiedene Vorrichtungen.

Bevorzugt ist wenigstens ein Ergebniswert charakteristisch für einen Betriebszustand und/oder Fehlerzustand der Vorrichtung und/oder einer Komponente dieser Vorrichtung und/oder einer mit der Vorrichtung verbundenen Komponente (etwa eine in Transportrichtung der Behältnisse stromaufwärts oder stromabwärts befindliche weitere Behandlungsvorrichtung).

Bevorzugt ist das Modell und/oder die Auswertevorrichtung dazu geeignet und bestimmt, durch Vornahme einer Mustererkennung aus der Vielzahl an Eingangsgrößen Muster zu erkennen, die zu einem von einem beabsichtigten Betriebszustand abweichenden Betriebszustand der Vorrichtung führen und/oder einen solchen bedingen bzw. verursachen und/oder bewirken.

Unter einem von einem beabsichtigten Betriebszustand abweichenden Betriebszustand wird insbesondere ein Betriebszustand der Vorrichtung verstanden, in welchem ein Fehlerzustand und/oder ein kritischer Betriebszustand, in welchem mit hoher (etwa vorgegebener) Wahrscheinlichkeit ein Fehlerzustand innerhalb eines vorgegebenen und/oder vorgebbaren Zeitraums auftreten wird, vorliegt. Unter einem von einem beabsichtigten Betriebszustand abweichenden Betriebszustand kann auch ein Betriebszustand verstanden werden, welcher zu unbeabsichtigten und/oder überdurchschnittlichen Abnutzungen und/oder über einer vorgegebenen und/oder vorgebbaren Höhe liegender Ressourcenverbrauch (etwa Energieverbrauch) führt und/oder solche bedingt.

Denkbar ist, dass - etwa durch einen Betreiber der Vorrichtung - vorgebbar und/oder vorgegeben ist, welche Betriebszustände der Kategorie der beabsichtigten Betriebszustände und welche Betriebszustände der Kategorie der unbeabsichtigten Betriebszustände zuzuordnen sind. Ein Betreiber könnte beispielsweise im Falle eines Auftretens seiner Ansicht nach nicht beabsichtigten Betriebszustands diesen Betriebszustand als nicht beabsichtigt kennzeichnen. Dies kann in Echtzeit (etwa von einer Überwachung der Vorrichtung) aber auch rückwirkend erfolgen (durch Kennzeichnung historischer Daten). Eine derartige Kennzeichnung kann mit Zeitstempel zusammen mit den in demselben Zeitraum erfassten Messwerten und weiteren Daten (etwa Betriebswerten) abgelegt und diesen zugeordnet werden.

Bevorzugt führt die Auswertevorrichtung mit einem ersten vorgegebenen Datensatz an Eingangsgrößen sowie damit jeweils zugeordneten für einen Betriebszustand der Vorrichtung charakteristischen Größe(n) und/oder jeweils zugeordneten Zeitstempeln in einer Mustererkennungsphase eine Mustererkennung durch, in welcher die Auswertevorrichtung Muster gewinnt.

Bevorzugt führt die Auswertevorrichtung in Abhängigkeit der gewonnenen Muster eine Klassifizierung durch, welche bevorzugt durch Zuordnung der (einem vorgegebenen Zeitraum zugeordneten) Eingangsgrößen in eine oder mehrere Klassen erfolgt. Die Klassen sind dabei bevorzugt charakteristisch für wenigstens einen Betriebszustand der Vorrichtung bzw. wenigstens einem, von dem beabsichtigten Betriebszustand abweichenden Betriebszustand.

Bevorzugt wird (während des Betriebs der Vorrichtung) bei Erkennung wenigstens eines durch die Mustererkennung gewonnenen Musters in den zugeführten Eingangsgrößen, welches zu einer vorgegebenen und/oder vorgebbaren Wahrscheinlichkeit zu einem von einem beabsichtigten Betriebszustand abweichenden Betriebszustand führt, eine Warngröße und/oder Benachrichtigungsgröße ermittelt und/oder zur Ausgabe (an den Betreiber der Vorrichtung) bereitgestellt und/oder (mittels einer Anzeigeeinrichtung) ausgegeben.

Denkbar ist, dass die Auswertevorrichtung auch nach einer ersten Mustergewinnungsphase fortlaufend und/oder in regelmäßig bzw. vorgegebenen und/oder vorgebbaren zeitlichen Abständen die ihr (bzw. dem Modell) zugeführten Daten (Eingangsgrößen) auf weitere und/oder neue (zu gewinnende) Muster hin analysiert bzw. auf eine Aktualisierung und/oder Verbesserung der Muster hin auswertet. Bevorzugt erfolgt die fortlaufende Mustererkennung bzw. Mustergewinnung zeitgleich mit der Identifizierung in aktuell in den Eingangsgrößen auftretender Muster. Bevorzugt ist die Auswertevorrichtung dazu geeignet und bestimmt, bevorzugt nach Bestätigung durch einen Bediener, neue und/oder abgeänderte (durch Mustererkennung gewonnene) Muster bei der Auswertung der zukünftig zugeführten Eingangsgrößen zu berücksichtigen und/oder bisherige Muster nicht mehr zu berücksichtigen.

Denkbar ist auch, dass als (weitere) Ausgabegröße(n) die zu dem erkannten Muster führenden wesentlichen Eingangsgröße(n) ermittelt und zur Ausgabe (an den Betreiber der Vorrichtung) bereitgestellt und/oder (mittels einer Anzeigeeinrichtung) ausgegeben werden. So könnten diese in besonderer Weise optisch hervorgehoben werden.

Bevorzugt gleicht nun, insbesondere auf Basis des vorhandenen Modells während dem Produktionslauf (oder in kurzen Pausen) die Anlage die Performance der Anlageenteile und insbesondere die physikalischen Eigenschaften der Kurvenrollen oder der Führungskurven an die Sollperformance insbesondere en an die Solleigenschaften dieser Kurvenrollen oder Führungskurven an.

Hierzu versucht bevorzugt die Behandlungseinrichtung, insbesondere die Erwärmungseinrichtung und/oder die Umformungseinrichtung und/oder die Etikettiereinrichtung, die beinflussbaren Produktionsdaten so einzustellen, dass die erwünschte Performance und/oder erreicht wird. Dabei kann insbesondere auch der Betrieb der Behältnisbehandlungsanlage an an einen Istzustand von Führungsrollen angepasst werden.

Bei Produktionsdaten wird bevorzugt zwischen drei verschieden Datenarten und/oder Parameterarten unterschieden, nämlich einerseits zwischen direkt beeinflussbaren Daten oder Parametern (z.B. Maschinengeschwindigkeit oder Fülldruck oder einer Heizleistung), indirekt beeinflussbaren Parametern (Wandstärkenverteilung, Preformtemperatur am Ofenauslauf oder Füllhöhe oder auch den Eigenschaften der gefertigen oder etikettierten Behältnissen und nicht beeinflussbaren Werten (Luftfeuchtigkeit, IR-Absorbtionsverhalten oder Hallentemperatur).

Um die gewünschte Sollperformance und/oder Sollzustände insbesondere im Hinblick auf einen Zustand der Kurvenrolle und/oder der Führungskurve zu erreichen kann es sein, dass verschieden kaskadierte Modelle genutzt werden. So ist es denkbar das es z.B. ein Modell gibt welches eine indirekt beinflussbare Größe (z.B. Wandstärkenverteilung, oder für den Tragring charakteristische Eigenschaften) über Anpassung von direkt beeinflussbaren Werten (z.B. Heizungsteller, Heizeinrichtungen oder Reckgeschwindigkeit oder Blasdrücke) derart anpasst, dass die im Hauptmodel hinterlegte Ausgestaltung des Tragrings zur aktuellen Hallentemperatur erzeugt wird, um die Sollperformance zu erreichen.

Ein weiterer Punkt, der bevorzugt in das Modell einfließen kann, sind Nebenbedingungen wie z.B. Energiebedarf oder Abfülldruck oder eine Kraft, mit der eine Blasdüse auf die zu expandierenden Kunststoffvorformlinge aufgesetzt wird. Es kann z.B. versucht werden der Sollperformance so nahe wie möglich zu kommen bei minimalem Energiebedarf.

Es ist auch denkbar, dass das Modell nach und nach geschärft wird, indem immer wieder Performancedaten erhoben werden und mit den Modellprognosedaten abgeglichen werden.

Die vorliegende Erfindung wird nunmehr an einem konkreten Beispiel nämlich einer Blasformmaschine erläutert. Derartige Blasformmaschinen weisen einen drehbaren Transportträger auf, an dem eine Vielzahl von Umformungsstationen angeordnet ist, welche Kunststoffvorformlinge zu Kunststoffbehältnissen umformen. Weiterhin weisen diese Maschinen bevorzugt auch Reckeinheiten auf, welche die Kunststoffvorformlinge in ihrer Längsrichtung dehnen. Bevorzugt weisen diese Maschinen auch Prozessregelungen auf, die insbesondere individuell für jede Umformungsstation die Umformungsprozesse regeln.

Daneben weisen diese Maschinen, wie oben erwähnt, bevorzugt Erwärmungseinrichtungen auf, welche die Kunststoffvorformlinge erwärmen.

Bevorzugt wird eine Bewegung der Reckstangeneinrichtung und/oder die Bewegung einer Blasdüseneinrichtung und/oder eine Öffnungs- und/oder Schließbewegung eine Blasform durch Führungskurven und an diesen Führungskurven abrollende Führungsrollen bewirkt.

Bereits bei der Prozessfindung bzw. Validierung der Eigenschaften der Führungsrollen und/oder Führungskurven werden bevorzugt mehrere rollen ausgebaut und im Nachgang Qualitätstests unterzogen. Dabei werden bevorzugt Optimierungsschleifen iterativ gefahren bis die Erkennungsqualität für gut befunden werden.

Stellt sich heraus, dass das beste Ergebnis bereits in den Anfängen dieser Optimierungsschleife gefunden wurde und im Nachgang trotz längerer Bemühungen keine weitere Verbesserung mehr gefunden werden konnte, würden gegebenenfalls auch diese ersten Ergebnisse als Kompromiss akzeptiert werden.

Im Folgenden werden unter Offlinemessungen solche Messungen an Führungskurven und/oder Führungsrollen verstanden, die außerhalb eines Herstellungsprozesses vorgenommen werden und die letztlich nachweisen sollen, ob eine Führungsrolle und/oder eine Führungskurve den geforderten Qualitätsansprüchen entspricht. Zu diesen Messungen zählen beispielsweise Kraftmessungen, Schwingungsmessungen, Geschwindigkeitsmessungen und dergleichen außerhalb eines Produktionsbetriebs.

Unter Onlinemessungen werden Messungen verstanden, die während der Produktion und insbesondere ohne eine Ausleitung von Behältnissen und/oder Kunststoffvorformlingen vorgenommen werden können (wie insbesondere Schwingungsmessungen im laufenden Betrieb, Kraftmessungen im laufenden Betrieb und dergleichen).

Korrelieren diese Messungen ausreichend gut mit den eigentlichen Qualitätsansprüchen, können Offlinemessungen reduziert werden oder völlig entfallen.

Bevorzugt werden jedoch, wie oben erwähnt auch Offlinemessungen und Onlinemessungen, welche hinsichtlich der gleichen Kurvenrolle durchgeführt wurden, einander zugeordnet.

Bei einem weiteren bevorzugten Verfahren werden sowohl Offlinemessungen vorgenommen, d. h. Messungen außerhalb des Arbeitsbetriebs, als auch Onlinemessungen, die im Produktionsbetrieb durchgeführt werden. Bevorzugt können Identifikationsinformationen sowohl für diese Offlinemessungen als auch für Onlinemessungen vorgegeben bzw. ausgegeben werden.

Die Identifikationsinformation bzw. die Ausleit-ID schafft die Möglichkeit, das Onlineregelziel exakt mit den "besten Offlinemessdaten" zu matchen. Aktuell werden die Sollwerte der Onlinemessdaten während der Einlernphase (DoE (statistische Versuchsplanung)) von dem Messsystem erfasst und schließlich dem Regelungssystem übergeben.

Dies sind bevorzugt nicht identisch diejenigen Werte, die im Validierungsprozess erreicht wurden, sondern "nur" diejenigen Onlinemesswerte, die sich bei der Wiederholung der besten Einstellparameter (Bestsetpoint-Einstellungen) aus dem Validierungsprozess nun in der Einlernphase (DoE) ergeben.

Da die auszuregelnden Störeinflüsse auch bei identischen Einstellparametern für eine Verschlechterung der Behältnisqualität sorgen können, besteht die Gefahr, dass bei der Einlernphase eben nicht die bestmögliche Behältnisqualität, sondern gegebenenfalls ein ungünstigeres Wanddickenprofil als Regelziel hinterlegt wird. Das Regelziel wäre somit schlechter als von einem Kunden gewünscht.

Falls man zu den ausgeleiteten Testrollen im Validierungsprozess jedoch die jeweilige Identifikationsinformation zur Verfügung hat, lassen sich aus der Datenbank allein darüber in Verbindung mit den zugehörigen Zeitstempeldaten nicht nur die kompletten Einstellparameter der Streckblasmaschine und/oder der Erwärmungseinrichtung (Best-setpoint-Einstellungen), sondern auch die zugehörigen Onlinemessdaten zu diesen Rollen als perfektes Regelziel wieder in die Messeinheit und in die Regelung übertragen.

Weiterhin ist es möglich, dass die gewonnenen Daten oder Messwerte für Vergleichszwecke und oder Analysezwecke verwendbar sind, insbesondere um auf unterschiedliche Maschinengrößen, Maschinenausführungen oder auch auf andere Maschinen schließen zu können, beispielsweise hinsichtlich Ersatzteillebensdauer und um Produktionsprognosen zu erhalten und um dadurch den Kundennutzen weiter steigern zu können.

Bei einer weiteren vorteilhaften Ausführungsform weist die Vorrichtung eine Vergleichseinrichtung auf, welche gemessene Messwerte, beispielsweise Schwingungswerte mit wenigstens einem Sollwert vergleicht und bevorzugt einen für diesen Vergleich charakteristischen Vergleichswert ausgibt. Bevorzugt wird damit ein Vergleich zwischen Ist-Werten beispielsweise einer Schwingungsmessung und Soll-Werten ausgegeben. Bevorzugt handelt es sich bei diesem Vergleichswert um eine Differenz oder einen Quotienten oder dergleichen. Besonders bevorzugt wird dieser Vergleichswert abgespeichert.

Bei einer vorteilhaften Ausführungsform weist die Vorrichtung eine Auswahleinrichtung auf, welche dazu geeignet und bestimmt ist, einen Sollwert aus einer Vielzahl von Sollwerten auszuwählen. Damit kann beispielsweise eine Vorauswahl eines Sollwerts getroffen werden, beispielsweise in Abhängigkeit von einem zu behandelnden Behältnis, einer Maschinengeschwindigkeit oder dergleichen. So kann beispielsweise eine Vielzahl von Sollwerten für Schwingungsmessungen in einer Speichereinrichtung abgelegt sein, welche jeweils unterschiedlichen Betriebsgeschwindigkeiten zugeordnet sind.

Bei einer weiteren vorteilhaften Ausführungsform weist die Vorrichtung wenigstens eine weitere Sensoreinrichtung auf, welche dazu geeignet und bestimmt ist, wenigstens einen für Einflussfaktoren charakteristischen Wert aufzunehmen. Bevorzugt ist dieser Wert aus einer Gruppe von Werten ausgewählt, welche Temperaturwerte, Druckwerte, Luftfeuchtigkeitswerte, Werte die für die zu behandelnden Behältnisse charakteristisch sind (etwa das Material des Behältnisses und/oder Kunststoffvorformlings oder eine Wandungsstärke oder ein Gewicht, Werte die beispielsweise für einen Etiketten - Klebstoff charakteristisch sind und dergleichen.

Besonders bevorzugt ist die Auswerteeinrichtung dazu geeignet und bestimmt, auch diese Werte mit zu berücksichtigen.

Bei einer weiteren vorteilhaften Ausführungsform weist die Erfassungseinrichtung ein Auswertemodul auf, welches dazu geeignet und bestimmt ist, Messwerte -insbesondere unter Berücksichtigung einer Ausgabe eines Drehgebers - auszuwerten, um die Messwerte den einzelnen Führungsrollen an einer Führungskurve oder auch Führungsrollen an unterschiedlichen Führungskurven zuzuordnen. Bevorzugt ist dabei dieser Drehgeber einem drehbaren Träger, an dem die einzelnen Behandlungseinrichtungen angeordnet sind, zugeordnet.

Bei einer weiteren vorteilhaften Ausführungsform ist die Zuordnung unter Berücksichtigung vorgegebener Daten durchführbar, Dabei kann die Zuordnung unter Berücksichtigung einer bestimmten Gruppe von Daten erfolgen oder auch unter Berücksichtigung unterschiedlicher Arten von Daten.

Bevorzugt sind die Daten aus einer Gruppe von Daten ausgewählt, welche einen Drehwinkel insbesondere eines Transportträgers (oder einer Behandlungseinrichtung) oder eine Position einer Transporteinrichtung, die Position einer Behandlungseinrichtung und/oder eine Zeitinformation, oder ein Signal eines Drehgebers enthält.

Bei dieser Ausgestaltung wird insbesondere bestimmt, zu welchem Zeitpunkt ein bestimmter Messwert ausgewertet wurde. Daneben kann anhand eines Drehgebers festgestellt werden, welche Behandlungsstation (und damit auch welche Kurvenrolle(n)) sich zu diesem Zeitpunkt an der Position der Sensoreinrichtung befunden hat.

Bei einer weiteren vorteilhaften Ausführungsform ist die Vorrichtung aus einer Gruppe von Vorrichtungen ausgewählt, welche Umformungseinrichtungen zum Umformen von Kunststoffvorformlingen zu Kunststoffbehältnissen, Fülleinrichtungen zum Befüllen von Behältnissen, Sterilisiereinrichtungen zum Sterilisieren von Behältnissen, Erwärmungseinrichtungen zum Erwärmen von Kunststoffvorformlingen, Druckeinrichtungen zum Bedrucken von Behältnissen, Etikettiereinrichtungen zum Etikettieren von Behältnissen, Verschließeinrichtungen zum Verschließen von Behältnissen mit Behältnisverschlüssen und Reinigungseinrichtungen zum Reinigen von Behältnissen enthält.

Bei einer weiteren vorteilhaften Ausführungsform weist die Erfassungseinrichtung wenigstens eine Sensoreinrichtung auf, welche wenigstens einen Messwert erfasst, der für wenigstens eine Bewegungsgröße der Führungsrolle oder der Behandlungseinrichtungen oder der Halteeinrichtung charakteristisch ist. Besonders bevorzugt ist diese Sensoreinrichtung aus einer Gruppe von Sensoreinrichtungen ausgewählt, welche Geschwindigkeitssensoren, Kraftsensoren, Beschleunigungssensoren, Schwingungssensoren und dergleichen enthält.

So kann beispielsweise aus dem Auftreten einer bestimmten Schwingung an einer Führungskurve auf einen Verschleiß einer an dieser Führungskurve abrollenden Führungsrolle (aber ggfs. auch auf einen Verschleiß der Führungskurve selbst) geschlossen werden.

Die vorliegende Erfindung ist weiterhin auf eine Behältnisbehandlungsanlage mit wenigstens einer Vorrichtung der oben bezeichneten Art gerichtet. Erfindungsgemäß weist die Behältnisbehandlungsanlage eine weitere Vorrichtung zum Behandeln von Behältnissen auf, welche bevorzugt dazu geeignet und bestimmt ist die Behältnisse in einer anderen Weise zu behandeln als die genannte Vorrichtung und es ist bevorzugt weiterhin eine (weitere) Erfassungseinrichtung vorgesehen, welche dazu geeignet und bestimmt ist, wenigstens einen für diese weitere Vorrichtung charakteristischen Messwert zu erfassen, wobei bevorzugt die Auswerteeinrichtung dazu geeignet und bestimmt ist, auch diesen weiteren Messwert bei der Auswertung zu berücksichtigen.

Bei dieser Ausführungsform wird berücksichtigt, dass oftmals auch Werte von anderen Maschinen einer Behältnisbehandlungsanlage die entsprechende Vorrichtung und die von der Erfassungseinrichtung erfassten Werte beeinflussen können. So können beispielsweise Werte einer Erwärmungseinrichtung für Kunststoffvorformlinge sich bei der eigentlichen Umformung der Behältnisse auswirken.

Bevorzugt sind die genannten Messwerte gemeinsam mit weiteren Maschinenparametern speicherbar. Besonders bevorzugt ist ein übergeordnetes Datenerfassungssystem vorgesehen, welches diese Messwerte verarbeitet und speichert.

Besonders bevorzugt ist dabei eine Verarbeitung an der Behältnisbehandlungsanlage bzw. der Maschine möglich. Besonders bevorzugt werden nur einzelne Datenpunkte übergeben.

Bei einer weiteren vorteilhaften Ausführungsform ist ein übergeordnetes System vorgesehen, in welchem die Daten verarbeitbar sind. Dabei ist es auch möglich, dass Daten oder Messwerte von mehreren Maschinen bzw. Vorrichtungen verarbeitet und analysiert werden.

Bei einer weiteren veränderten Ausführungsform ist die genannte Erfassungseinrichtung an der Führungskurve derart angeordnet, dass die Erfassungseinrichtung die Laufeigenschaft für jede einzelne Rolle selektiv erfassen kann, wobei diese einzelnen Rollen an der Führungskurve abrollen.

Bei einer weiteren vorteilhaften Ausführungsform weist die Führungskurve einen ersten Führungskurvenbereich sowie einen zweiten Führungskurvenbereich auf. Vorteilhaft sind dieser erste und der zweite Führungskurvenbereich voneinander (mechanisch) entkoppelt. Besonders bevorzugt ist dabei ein Sensor der Erfassungseinrichtung in einem der beiden Führungskurvenbereiche und insbesondere in dem zweiten (oder ersten) Führungskurvenbereich angeordnet.

Bei einer weiteren bevorzugten Ausführungsform weist die Erfassungseinrichtung eine Erfassungsstrecke auf, die einen ersten Führungskurvenbereich und einen zweiten Führungskurvenbereich miteinander (mechanisch) koppelt wobei bevorzugt in dem zweiten Führungskurvenbereich eine Sensoreinrichtung der Erfassungseinrichtung zur Erfassung von Schwingungen angeordnet ist und insbesondere zum Erfassen von denjenigen Schwingungen vorgesehen ist, die von einer Führungsrolle in die Erfassungsstrecke injiziert werden.

Besonders bevorzugt sind der erste Führungskurvenbereich und der zweite Führungskurvenbereich durch eine Aussparung und oder einen Spalt voneinander entkoppelt. Besonders bevorzugt sind der erste Führungskurvenbereich und der zweite Führungskurvenbereich lediglich im Bereich einer Erfassungsstrecke miteinander verbunden.

Bei einer weiteren vorteilhaften Ausführungsform ist die erfasste Strecke derart ausgestaltet, dass der zweite Führungskurvenbereich einer vorbestimmten Erfassungsrichtung relativ zu dem Führungskurvenbereich schwingt, wenn auf der Erfassungsstrecke eine Rolle abholt.

Besonders bevorzugt ist die Erfassungsstrecke gegenüber Schwingungen unempfindlich, die in einem übrigen Rollweg der Führungskurve injiziert werden. Auf diese Weise ist es möglich, dass die Erfassungseinrichtung gezielt auftretende Schwingungen einer bestimmten Führungsrolle zuordnen kann. Dieser folgt bevorzugt auch durch Auswertung von Signalen eines Drehgebers.

Bei einer weiteren vorteilhaften Ausführungsform weist diese Strecke eine Länge auf, die mindestens einem Außenumfang einer Führungsrolle entspricht, die auf der Strecke abholt. Auf diese Weise ist eine genaue Zuordnung zu einer bestimmten Führungsrolle und damit bevorzugt auch zu einer bestimmten Behandlungseinrichtungen möglich.

Bei einer weiteren vorteilhaften Ausführungsform ist die vorbestimmte Erfassungseinrichtung senkrecht zu einer Ebene angeordnet, in welcher die Führungsrollen an der Erfassungsstrecke abrollen.

Bei einer weiteren vorteilhaften Ausführungsform ist die Ausdehnung des zweiten Führungskurvenbereichs in der Richtung der Länge der Erfassungsstrecke größer als die Länge der Erfassungsstrecke.

Bei einer weiteren vorteilhaften Ausführungsform ist hier Erfassungseinrichtung als Sensor ausgestaltet, welcher einen Beschleunigungssensor aufweist, der insbesondere zum Erfassen von Beschleunigungsamplituden geeignet und bestimmt ist.

Bei einem bevorzugten Verfahren handelt es sich bei der Auswerteeinrichtung um eine externe Auswerteeinrichtung, insbesondere einen Cloud-basierten und/oder einen externen Server. Unter einem externen Server ist insbesondere ein in Bezug auf die Vorrichtung ein externer Server, insbesondere ein Backend-Server, zu verstehen. Der externe Server ist beispielsweise ein Backend eines Herstellers der Vorrichtung oder eines Dienstanbieters, welcher dazu eingerichtet ist, Messwerte und/oder hiervon abgeleitete Daten und/oder Fehlerzustände der Vorrichtung und/oder Betriebszustände der Vorrichtungen auszuwerten und/oder zu verwalten und/oder zu speichern. Die Funktionen des Backend bzw. des externen Servers können dabei auf (externen) Serverfarmen durchgeführt werden. Beim (externen) Server kann es sich um ein verteiltes System handeln. Der externe Server und/oder das Backend kann Cloud-basiert sein.

Die vorliegende Erfindung ist weiterhin auf ein Verfahren zum Behandeln von Behältnissen gerichtet, wobei eine Transporteinrichtung die zu behandelnden Behältnisse entlang eines vorgegebenen Transportpfads transportiert und wenigstens eine Behandlungseinrichtung wenigstens ein Behältnis und bevorzugt ein von der Transporteinrichtung transportiertes Behältnis in einer vorgegebenen Weise behandelt, wobei die Vorrichtung eine Bewegungseinrichtung aufweist, welche bevorzugt eine Relativbewegung zwischen dem zu behandelnden Behältnis und der Behandlungseinrichtung in einer Bewegungsrichtung bewirkt.

Weiterhin weist diese Bewegungseinrichtung wenigstens eine erste Führungskurve auf, sowie eine Führungsrolle, welche gegenüber der ersten Führungskurve abrollt. Weiterhin weist die Vorrichtung eine Erfassungseinrichtung auf, welche wenigstens einen Messwert erfasst, der für eine Laufeigenschaft der Führungsrolle gegenüber der Führungskurve charakteristisch ist.

Erfindungsgemäß weist die Vorrichtung eine Auswerteeinrichtung auf, welche diesen erfassten Messwert unter Berücksichtigung wenigstens eines weiteren für den Betrieb der Vorrichtung charakteristischen Wertes auswertet.

Besonders bevorzugt wird ein weiterer für den Behandlungsvorgang charakteristischer Parameter oder Wert erfasst und bevorzugt dieser weitere Parameter bei der Auswertung berücksichtigt.

Bei diesem weiteren charakteristischen Wert kann es sich beispielsweise um eine Temperatur einen Druck, eine Luftfeuchtigkeit oder dergleichen handeln.

Besonders bevorzugt wird der für den Betrieb der Vorrichtung charakteristische Wert und/oder der weitere charakteristische Wert oder Parameter bei der Ermittlung von Sollwerten berücksichtigt.

Weitere Vorteile und Ausführungsformen ergeben sich aus den beigefügten Zeichnungen.

Darin zeigen:
- Fig. 1: eine stark vereinfachte Draufsicht auf eine Behälterbehandlungsanlage gemäß einem Ausführungsbeispiel;
- Fig. 2: eine Seitenansicht einer sehr schematisch dargestellten Halterung für Behältnisse an einer Führungskurve mit einer Erfassungseinrichtung der Vorrichtung gemäß dem Ausführungsbeispiel;
- Fig. 3: eine Draufsicht auf die sehr schematisch dargestellte Halterung für Behälnisse an der Führungskurve der Vorrichtung gemäß dem Ausführungsbeispiel;
- Fig. 4: eine vereinfachte Teildraufsicht auf die Führungskurve mit der Erfassungseinrichtung der Vorrichtung gemäß dem Ausführungsbeispiel;
- Fig. 5: eine weitere vereinfachte Teildraufsicht auf die Führungskurve mit der Erfassungseinrichtung der Vorrichtung gemäß dem Ausführungsbeispiel;
- Fig. 6: eine Darstellung der Empfindlichkeit einer Erfassungsstrecke der Erfassungseinrichtung gemäß dem Ausführungsbeispiel für verschiedenen Frequenzen;
- Fig. 7: ein schematisches Bockschaltbild der Erfassungseinrichtung gemäß dem Ausführungsbeispiel;
- Fig. 8 - 10: jeweils ein Frequenzspektrum von Erfassungsergebnissen an verschiedenen Stellen der Erfassungsstrecke der Erfassungseinrichtung gemäß dem Ausführungsbeispiel;
- Fig. 11: eine weitere vereinfachte Teildraufsicht auf die Führungskurve mit der Erfassungseinrichtung der Behälterbehandlungsanlage gemäß dem Ausführungsbeispiel;
- Fig. 12,13: jeweils ein Auswertediagramm der Erfassungseinrichtung der Behälterbehandlungsanlage gemäß dem Ausführungsbeispiel;
- Fig. 14: eine Seitenansicht einer Führungskurve;
- Fig. 15: eine Draufsicht der in Fig. 14 gezeigten; und
- Fig. 16: eine Detailansicht der Führungskurve aus Fig. 15.

In den Figuren sind gleiche oder funktionsgleiche Elemente, sofern nichts anderes angegeben ist, mit denselben Bezugszeichen versehen.

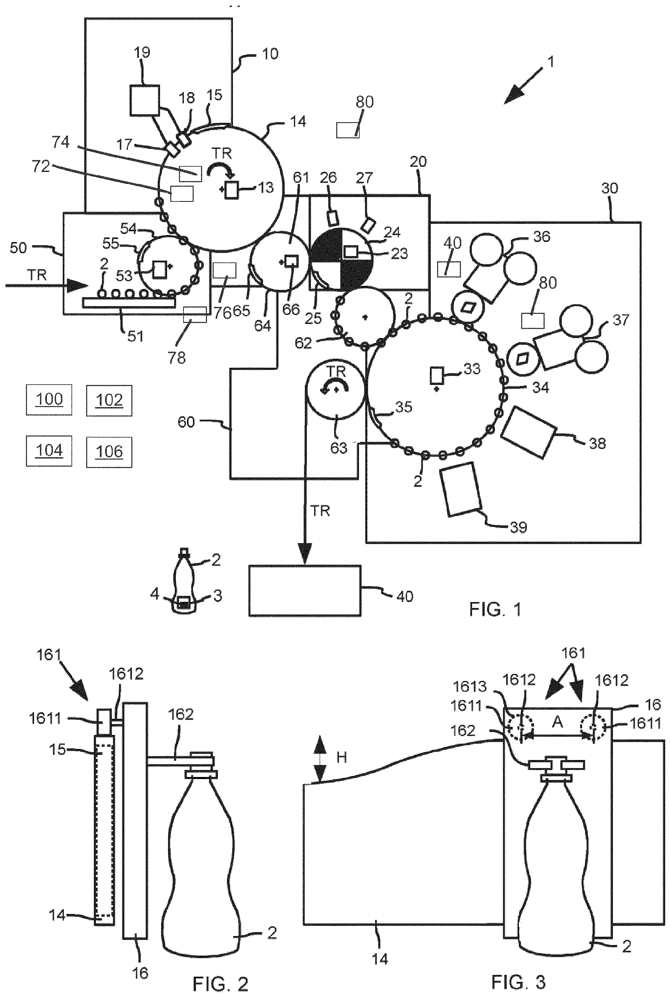

Fig. 1 zeigt eine Behälterbehandlungsanlage 1 zur Behandlung von Behältnissen 2, im Folgenden auch als Behälter bezeichnet, die Flaschen, Dosen, Kästen, Gebinde, Fässer usw. sein können, und die bevorzugt mit einem sehr schematisch dargestellten Etikett 3 und/oder einer beliebigen Bedruckung 4 als Kennzeichnung ausstattbar sind.

Hierfür weist die Behälterbehandlungsanlage 1 als ein Beispiel eine erste bis vierte Vorrichtungen 10, 20, 30, 40 zum Behandeln von Behältnissen (im Folgenden kurz als Vorrichtungen bezeichnet) auf, die bevorzugt miteinander gekoppelt und/oder synchronisiert sind. Die Behälterbehandlungsanlage 1 kann jedoch auch nur eine oder zwei oder drei der Vorrichtungen 10, 20, 30, 40 aufweisen. Es können auch mehr Vorrichtungen 10, 20, 30, 40 vorhanden sein. Demzufolge ist jede beliebige Zahl von Vorrichtungen 10, 20, 30, 40 möglich. In Fig. 1 sind der Einfachheit halber nicht alle Behältnisse 2 dargestellt oder mit einem Bezugszeichen versehen.

Diese einzelnen Vorrichtungen 10, 20, 30, 40 weisen bevorzugt jeweils (nicht mit eigenen Bezugszeichen bezeichnete) Transporteinrichtungen auf, welche die Behältnisse während deren Behandlung transportieren. Diese Transporteinrichtungen weisen bevorzugt jeweils Transportsterne oder Transporträder auf, welche mit Halteeinrichtungen zum Halten der Behältnisse versehen sind.

Bei wenigstens einer der Vorrichtungen 10, 20, 30, 40 handelt es sich um erfindungsgemäße Vorrichtungen. Bevorzugt handelt es sich bei mehreren der Vorrichtungen 10, 20, 30, 40 um erfindungsgemäße Vorrichtungen und besonders bevorzugt handelt es sich bei allen der Vorrichtungen 10, 20, 30, 40 um erfindungsgemäße Vorrichtungen.

Ein Behältnis 2 kann beispielsweise ein Behältnis sein, das ein Fassungsvermögen von maximal ca. 0,33 Liter oder 0,5 Liter oder 1,5 Liter usw. hat. Daneben sind auch andere Fassungsvermögen denkbar. Hierbei kann die Form des Behälters 2 frei gewählt werden. Außerdem kann das Material des Behälters 2 frei gewählt werden, wie beispielsweise Glas, Kunststoff, insbesondere PET, Aluminium, etc.

Im Zulauf der ersten Vorrichtung 10 ist eine Transporteinrichtung 50 vorgesehen, welche mit Hilfe einer Transportschiene 51 und einer Führungskurve 54, im Folgenden auch als Hubkurve bezeichnet der ersten Vorrichtung 10 Behältnisse 2 aufgereiht in einer Reihe oder als Behältnisstrom zuführt. Die Führungskurve 54 bildet eine Rundläufertransporteinrichtung, die zudem bevorzugt einen Drehgeber 53 aufweist. Die Führungskurve 54 ist außerdem bevorzugt mit einer Erfassungseinrichtung 55 versehen.

Das Bezugszeichen 100 bezieht sich auf eine Auswerteeinrichtung, welche, wie unten genauer beschrieben wird, dazu geeignet und bestimmt ist, Messwerte der Erfassungseinrichtung(en) auszuwerten, wobei im Rahmen dieser Auswertung auch weitere für den Betrieb einer oder mehrerer Vorrichtungen zum Behandeln von Behältnissen charakteristische Werte berücksichtigt werden, wie etwa Werte von einzelnen oder mehreren Sensoren einer Etikettiereinrichtung, wie unten genauer beschrieben.

Das Bezugszeichen 102 kennzeichnet eine Zuordnungseinrichtung, welche dem oder den Messwerten einen Betriebswert zuordnet, der für den Betrieb der jeweiligen Vorrichtung oder der gesamten Behältnisbehandlungsanlage charakteristisch ist. Dabei kann es sich um einen Betriebswert handeln, der von derselben Vorrichtung stammt, an der auch der Messwert gemessen wird oder von einer anderen der Vorrichtungen. Daneben kann es sich bei dem Betriebswert auch um einen Umgebungswert handeln wie eine Umgebungstemperatur oder um einen Betriebswert, der für die gesamte Behältnisbehandlungsanlage charakteristisch ist, wie etwa eine Produktionsgeschwindigkeit. Bevorzugt wird dabei dieser Betriebswert.

Insbesondere ist dabei dieser Betriebswert auch mit einer zeitlichen Zuordnung abspeicherbar, d.h. er wird bevorzugt zu dem Zeitpunkt oder in dem Zeitraum erfasst, zu dem auch der Messwert erfasst wurde.

Bevorzugt ist weiterhin eine Speichereinrichtung 104 vorgesehen, in denen eine Vielzahl von Messwerten, insbesondere mit den diesen Messwerten zugeordneten Betriebswerten und bevorzugt auch mit einem für einen Zeitpunkt des Auftretens dieser Werte abspeicherbar ist.

Das Bezugszeichen 106 kennzeichnet eine Vergleichseinrichtung, welche dazu geeignet und bestimmt ist, aufgenommene Messwerte mit Sollwerten zu vergleichen. Dabei werden bevorzugt diese Sollwerte von einer Auswahleinrichtung aus einer Vielzahl von Sollwerten ausgewählt. Diese Auswahl erfolgt besonders bevorzugt unter Verwendung einer künstlichen Intelligenz.

Die erste Vorrichtung 10 ist bevorzugt als Rundläufermaschine mit einer Führungskurve 14 ausgeführt, bei der bevorzugt eine Erfassungseinrichtung 15 vorgesehen ist.

Zudem hat die zweite Vorrichtung 20 bei dem Beispiel von einen Fig. 1 Drehgeber 23 und eine Führungskurve 24 mit einer Erfassungseinrichtung 25. Außerdem weist bevorzugt die dritte Behälterbehandlungsmaschine 20 bei dem
Beispiel einen Drehgeber 33 und eine Führungskurve 34 mit einer Erfassungseinrichtung 35 auf.

Die Transportrichtung TR der Behältnisse 2 ist in jeweils durch Pfeile angegeben und ergibt sich auch aus der Reihe von Behältern 2 entlang der Vorrichtungen 10, 20, 30, 40.

Zwischen der dritten und vierten Vorrichtung 30, 40 werden die Behältnisse 2 mit einer Transporteinrichtung 60 mit Hilfe von Transportmodulen 61, 62 , 63 transportiert. Das Transportmodul 61 hat eine Führungskurve 64, bei der eine Erfassungseinrichtung 65 und ein Drehgeber 66 vorgesehen sind.

Diese Transportmodule sind bevorzugt als Transportsterne ausgeführt, welche einen drehbaren Träger aufweisen, an dem eine Vielzahl von Halteeinrichtungen zum Halten der Kunststoffvorformlinge bzw. Kunststoffbehältnisse angeordnet ist. Bei einer bevorzugten Ausführungsform ist wenigstens eines dieser Transportmodule als Teilungsverzugsstern ausgebildet, welcher dazu geeignet und bestimmt ist, eine Teilung bzw. einen Abstand aufeinanderfolgend transportierter Kunststoffvorformlinge oder Kunststoffbehältnisse zu verändern.

Ansonsten weisen die Transporteinrichtungen 50, 60 bei dem Beispiel in Fig. 1 Multifunktionssterne oder Karussells zum Transport der Behältnisse 2 auf. Es ist auch möglich, dass mindestens eine der Vorrichtungen 10, 20, 30, 40 alternativ als Multifunktionsstern oder Karussell ausgebildet ist oder ein solches aufweist.

Bei dem Beispiel von Fig. 1 bilden die Vorrichtungen 10, 20, 30, 40 und die Transporteinrichtungen 50, 60 einen Block, der sehr kompakt aufgebaut ist. Auch die vierte Vorrichtung 40 kann hierbei in der Nähe oder auch angrenzend am Block aus den Vorrichtungen 10, 20, 30 und den Transporteinrichtungen 50, 60 angeordnet sein, auch wenn dies in nicht dargestellt ist.

Die Behälterbehandlungsanlage 1 und die Vorrichtungen 10, 20, 30, 40 sind jedoch nicht darauf beschränkt und können auch in separater Ausführung aneinandergereiht sein.

Bei dem Beispiel von Fig. 1 ist die erste Vorrichtung 10 eine Füllmaschine, in welcher die Behältnisse 2 zu sehr schematisch dargestellten Füllventilen 17, 18 bewegt werden, mit welchen aus mindestens einem Fülltank mindestens ein Produkt in die von der Transporteinrichtung 50 zugeführten Behältnisse 2 abgefüllt wird . Bevorzugt bewegen sich die Füllventile mit den Behältnissen mit. Das Produkt kann beispielsweise ein Lebensmittel, insbesondere ein Getränk usw., oder ein Reinigungsmittel oder Kosmetikprodukt oder ein Pulver oder Stückgut, insbesondere Kügelchen, usw., oder irgendein sonstiges Produkt sein, das in Behälter 2 abfüllbar ist.

An dem Fülltank 19 kann auch ein Sensor 72 zum Messen des Füllstands des Produkts im Fülltank 19 vorgesehen sein. Das Bezugszeichen 74 kennzeichnet einen weiteren Sensor, der zur Erfassung einer Ventilstellung eines Füllventils geeignet und bestimmt ist.

Bevorzugt können die von diesen Sensoreinrichtungen 72, 74 ausgegebenen Werte oder Daten verwendet werden um von der Auswerteeinrichtung als weitere für den Betrieb der Vorrichtung charakteristische Werte verwendet zu werden. Bevorzugt kann dieser Fülltank jedoch auch an einem drehbeweglichen Träger angeordnet sein und sich so mitdrehen.

Bei dem Beispiel von Fig. 1 ist die zweite Behälterbehandlungsmaschine 20 ein Verschließer, welcher der Füllmaschine oder ersten Behälterbehandlungsmaschine 10 nachgeschaltet ist. In dem Verschliesser als zweite Behälterbehandlungsmaschine 20 werden die Behälter 2 mit Hilfe von beispielsweise mindestens einer Schraubantriebseinrichtung 26, 27 mit einer Verschlusskappe versehen und dadurch verschlossen. Zur Vereinfachung sind weitere Komponenten der zweiten Behälterbehandlungsmaschine 20 nicht dargestellt und beschrieben.

Das Bezugszeichen 76 kennzeichnet einen weiteren Sensor, der wenigstens einen Wert erfasst, der für einen Veschliessvorgang der Behältnisse charakteristisch ist. Dies kann etwa eine Drehstellung eines Verschlusses gegenüber einem zu verschließenden Behältnis sein. Bevorzugt können auch von diesem weiteren Sensor ausgegebene Werte als weitere Werte von der Auswerteeinrichtung zur Auswertung herangezogen werden.

Bei dem Beispiel von Fig. 1 ist die dritte Vorrichtung 30 eine Ausstattungsmaschine, welche die von der Füllmaschine oder ersten Vorrichtung 10 gefüllten Behälter 2 und dann mit der zweiten Vorrichtung 20 geschlossenen Behälter 2 mit einer Kennzeichnung ausstattet durch beispielsweise das Etikett 3 und/oder eine beliebige Bedruckung 4. Hierfür hat die Ausstattungsmaschine bei dem Beispiel von Fig. 1 die Führungskurve 34. An der Führungskurve 34 werden die Behälter 2 mittels Halterungen vorbei an vier verschiedenen Modulen geführt, die insbesondere Ausstattungsmodule 36, 37, 38, 39 sind. Beispielweise ist das Ausstattungsmodul 36 ein Etikettier- und/oder Druckaggregat, das Ausstattungsmodul 37 ist ein Etikettieraggregat und die Ausstattungsmodule 38, 39 sind als Druckaggregate ausgebildet. Demzufolge kann das Etikett 3 ein vorgefertigtes Etikett sein. Auch kann das Etikett ein Selbstklebeetikett sein.

Das Bezugszeichen 78 kennzeichnet einen Sensor, der dazu geeignet und bestimmt ist, wenigstens einen für den Ausstattungsvorgang charakteristischen Wert zu erfassen, wie etwa eine Temperatur eines Ettikettierleims oder eine Drehzahl eines Etikettierzylinders. Bevorzugt können auch von diesem weiteren Sensor ausgegebene Werte als weitere Werte von der Auswerteeinrichtung zur Auswertung herangezogen werden.

Jedoch kann das Etikett 3 auch erst mittels eines Etikettier- und/oder Druckaggregats zumindest teilweise gedruckt werden, was durch die Bedruckung 4 veranschaulicht ist. Alternativ kann das Etikettier- und/oder Druckaggregat auch zumindest teilweise direkt auf das Etikett 3 oder den Behälter 2 drucken. Insbesondere kann mit dem Etikettier- und/oder Druckaggregat oder den Druckaggregaten mit einem Drucker, wie einem Tintenstrahl- oder Laserdrucker, eine zusätzliche Kennzeichnung auf die Behälter 2 gedruckt werden, um eine Rückverfolgung der Behälter 2 oder Produkte sicherzustellen.

Alternativ können die Ausstattungsmodule 35 bis 38 jedoch jeweils gleich ausgestaltet sein, so dass alle Behälter 2 mit der gleichen Sorte von Kennzeichnungen, insbesondere Etiketten 3, versehen werden. Bei dem in Fig. 1 gezeigten Fall können die Behälter 2 insbesondere mit verschiedenen Etikettensorten, wie beispielsweise Bauch und/oder Brustetikett und/oder Rundumetikett von der Rolle oder als Blatt und/oder Selbstklebeetikett und/oder Kaltleimetikett und/oder Heißleimetikett usw. versehen werden. Es sind beliebige Kombinationen von Ausstattungsmodulen 35 bis 38 und damit Sorten von Kennzeichnungen, insbesondere Etikettensorten, denkbar und realisierbar.

Zur Vereinfachung sind weitere Komponenten der dritten Vorrichtung 30, wie insbesondere Druckköpfe, Sensoren, Antriebseinrichtungen für Etikettenrollen, usw. nicht dargestellt und beschrieben.

Nach der dritten Vorrichtung 30, der Ausstattungsmaschine bei dem zuvor gewählten Beispiel, werden die Behälter 2 mit Hilfe der Transporteinrichtung 60 der vierten Vorrichtung 40 zugeführt, wie bereits zuvor erwähnt.

Die vierte Vorrichtung 40 ist beispielsweise eine Verpackungsmaschine, bei welcher die Behälter 2 in vorbestimmte Sorten und/oder Verpackungsgrößen, verpackt werden, beispielsweise als Gebinde mit zwei Behältern 2 oder vier Behältern 2, mit oder ohne Tragegriff usw., oder in eine Kiste oder Karton, usw. Zum Herstellen von beispielsweise Gebinden oder auch für die Kartons kann eine Schrumpfstation vorgesehen sein. Auch bei der Verpackungsmaschine kann eine Hubkurve bzw. Führungskurve mit Erfassungseinrichtung vorgesehen sein, auch wenn dies in Fig. 1 nicht gezeigt ist.

Auch die Vorrichtung 40 weist bevorzugt eine oder mehrere Sensoreinrichtungen auf, welche für den Betrieb dieser Vorrichtung charakteristische Werte aufnimmt. Dies kann beispielsweise ein Wert sein, der für den Verpackungsvorgang charakteristisch ist wie etwa eine Temperatur eines Schrumpftunnels. Bevorzugt können auch von diesem weiteren Sensor ausgegebene Werte als weitere Werte von der Auswerteeinrichtung zur Auswertung herangezogen werden.

Das Bezugszeichen 80 kennzeichnet grob schematisch eine Sensoreinrichtung, welche dazu geeignet und bestimmt sind, Umgebungswerte aufzunehmen, wie beispielsweise eine Umgebungstemperatur, einen Umgebungsdruck und dergleichen. Dabei wäre es auch möglich, dass jeder der Vorrichtungen 10, 20, 30, 40 eine derartige Sensoreinrichtung zugeordnet ist, welche (insbesondere lokal auf diese Vorrichtung bezogen diese Umgebungswerte aufnimmt.

Bevorzugt können auch von dieser Sensoreinrichtung ausgegebene Werte als weitere Werte von der Auswerteeinrichtung zur Auswertung herangezogen werden.

Die zuvor beschriebenen Füllventile 17, 18, die Antriebseinrichtungen 26, 27, die Module 36 bis 39 und nachfolgend beschriebene Halteklammern sind Behandlungselemente 17, 18, 26, 27, 36 bis 39, 162 der Behälterbehandlungsanlage 1 bzw. ihrer jeweiligen Vorrichtungen 10, 20, 30, 40.

Obenstehend wurde die Erfindung anhand einer Behältnisbehandlungsanlage erläutert, welche welche ein Fülleinrichtung, eine Verschließeinrichtung, eine Etikettiereinrichtung und eine Verpackungseinrichtung als Vorrichtungen zum Behandeln von Behältnissen aufweist.

Die Erfindung ist jedoch auch auf andere Behältnisbehandlungsanlagen anwendbar beispielsweise eine Anlage, welche eine Vorrichtung zum Erwärmen von Kunststoffvorformlingen, eine Umformungseinrichtung zum Umformen von Kunststoffvorformlinge zu Kunststoffbehältnissen, eine Sterilisationseinrichtung zum Sterilisieren von Kunststoffvorformlingen und/oder von Kunststoffbehältnissen und/oder eine Inspektionseinrichtung zum Inspizieren von Behältnissen aufweist.

Dabei können Sensoren vorgesehen sein, welche andere Werte erfassen, wie etwa Positinen von Reckstangen, Blasdrücke, Heizleistungen von Heizelementen der Erwärmungseinrichtung und dergleichen.

Fig. 2 zeigt als Beispiel eine stark vereinfachte Schnittansicht der Führungskurve bzw. Hubkurve 14 mit der Erfassungseinrichtung 15. Die Führungskurven 24, 34, 54, 64 mit den jeweils zugehörigen Erfassungseinrichtungen 25, 35, 55, 65 sind bevorzugt auf die gleiche Weise ausgeführt, so dass die nachfolgende Beschreibung der Führungskurve 14 und der Erfassungseinrichtung 15 auch für die Führungskurven 24, 34, 54, 64 mit den jeweils zugehörigen Erfassungseinrichtungen 25, 35, 55, 65 gilt.

An der Führungskurve 14 ist eine Halterung 16 zum Transport eines Behältnisses 2 entlang der Führungskurve 14 vorgesehen. Die Halterung 16 hat mindestens eine (Führungs)Rolle 161, welche an der Führungskurve 14 abrollt. Genauer gesagt rollt sich bei dem Beispiel von Fig. 2 ein Wälzkörper 1611 der mindestens einen (Führungs)Rolle 161 oben auf der Führungskurve 14 ab. Hierfür ist bevorzugt der Wälzkörper 1611 über eine Achse 1612 drehbar an der Halterung 16 befestigt. Zudem hat bevorzugt die Halterung 16 eine Halteeinrichtung und insbesondere eine Halteklammer 162 zum Halten des Behältnisses 2. Die Halterung 16 kann beispielsweise mittels nicht dargestellten Druckfedern oder anderweitig nach unten an der Führungskurve 14 vorgespannt sein, so dass die mindestens eine (Führungs)Rolle 161 sicher an der Führungskurve 14 läuft.

Wie auch aus Fig. 3 in einer Draufsicht auf einen anderen Abschnitt der Führungskurve 14 ersichtlich, läuft an der Führungskurve 14 in der Regel eine Vielzahl von Rollen 161 gleichzeitig. Die (Führungs)Rollen 161 rollen mit dem Außenumfang 1613 des Wälzkörpers 1611
Die (Führungs)kurve 14 ist bevorzugt derart geformt, dass die Halterung 16 in der Höhe H verstellbar ist. Alternativ ist es beispielsweise möglich, beispielsweise eine Zentrierglocke, die zum Zentrieren der Behälter 2 an einem Drehteller dient, in der Höhe zu verstellen. Es sind beliebige andere Anwendungen für die Führungskurve 14 denkbar.

Die (Führungs)Rollen 161 injizieren jeweils Schwingungen in die Führungskurve 14. Die Schwingungen sind mit der Erfassungseinrichtung 15 als Körperschall bzw. Beschleunigung erfassbar. Damit der Körperschall nicht nur als breitbandiges Rauschen wahrnehmbar ist, sind die Führungskurve 14 und die Erfassungseinrichtung 15 ausgestaltet, wie nachfolgend beschrieben. Dadurch ist es möglich, mit der Erfassungseinrichtung 15 die Laufeigenschaften einer einzelnen (Führungs)Rolle 161 auf der Führungskurve 14 zu erfassen.

Die Führungskurve 14, wie auch jede andere der zuvor genannten Führungskurven, kann alternativ segmentförmig ausgestaltet sein. In diesem Fall laufen die Rollen 161 nicht auf einem durch die Führungskurve 14 geschlossenen Kreis sondern nur auf einem vorbestimmten Kreissegment. Die Führungskurve 14, wie auch jede andere der zuvor genannten Führungskurven, kann alternativ oder zusätzlich zumindest teilweise einen linearen oder ovalen Abschnitt aufweisen, wenn dies für den jeweiligen Anwendungsfall geeignet erscheint.

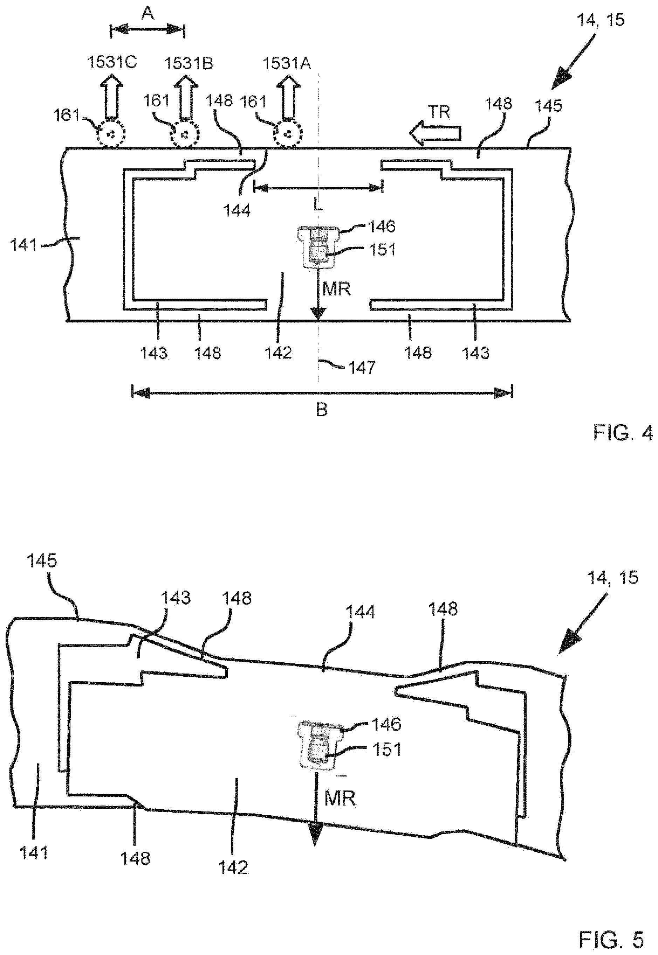

Gemäß Fig. 4 ist die Erfassungseinrichtung 15 teilweise in die Führungskurve 14 integriert, die hierfür speziell ausgestaltet ist. Die Führungskurve 14, die in Fig. 4 nur teilweise dargestellt ist, hat einen ersten Führungskurvenbereich 141, welcher bei dem Beispiel von als Kreissegment dargestellt ist.

Die Führungskurve 14 kann jedoch einen Vollkreis bilden, der den ersten Führungskurvenbereich 141 umfasst. Zudem hat die Führungskurve 14 einen zweiten Führungskurvenbereich 142, der bei dem
Beispiel von einteilig mit dem ersten Führungskurvenbereich 141 ausgestaltet ist. Der erste und zweite Führungskurvenbereich 141, 142 sind durch eine erste Aussparung 143 teilweise entkoppelt voneinander. Der erste und zweite Führungskurvenbereich 141, 142 sind nur im Bereich einer Erfassungsstrecke 144 miteinander verbunden und dadurch miteinander gekoppelt.

Die Erfassungsstrecke 144 mit einer Länge L ist entlang eines Rollwegs 145 der mindestens einen Rolle 161 an der Führungskurve 14 vorgesehen. Bei dem Beispiel von Fig. 4 ist der Rollweg 145 oben auf der Führungskurve 14 angeordnet. In einer zweiten Aussparung 146 des zweiten Führungskurvenbereichs 142 ist ein Sensor 151 der Erfassungseinrichtung 15 angeordnet.

Die erste und zweite Aussparung 143, 146 sind in der Laufrichtung der Rolle 161 auf dem Rollweg 145 jeweils symmetrisch zu einer Symmetrielinie 147 der Erfassungsstrecke 144 angeordnet. Die Symmetrielinie 147 ist außerdem die Symmetrielinie des zweiten Führungskurvenbereichs 142. Somit ist die zweite Aussparung 143 in der Mitte des zweiten Führungskurvenbereichs 142 angeordnet. Der zweite Führungskurvenbereich 142 hat in Laufrichtung der mindestens einen Rolle 161 eine maximale Ausdehnung B.

Die maximale Ausdehnung B des zweiten Führungskurvenbereichs 142 in der Richtung der Länge L der Erfassungsstrecke 144 ist größer als die Länge L der Erfassungsstrecke 144 . Zudem ist die Aussparung 143 in dem Bereich senkrecht zur Erfassungsrichtung MR bzw. parallel zu Rollweg 145 und zur Erfassungsstrecke 144 stufenförmig ausgeführt. Bei dem Beispiel von Fig. 4 hat der zweite Führungskurvenbereich 142 ausgehend von der Symmetrielinie 147 jeweils zwei Stufen bis zu einem Teil der Aussparung 143, der in etwa parallel zu der Symmetrielinie 147 angeordnet ist. Der zweite Führungskurvenbereich 142 ist in seiner Mitte bei der Symmetrielinie 147 breiter als an seinem Enden. Die genaue Ausgestaltung des Führungskurvenbereichs 142 ist im Rahmen der folgenden Bedingungen frei wählbar.

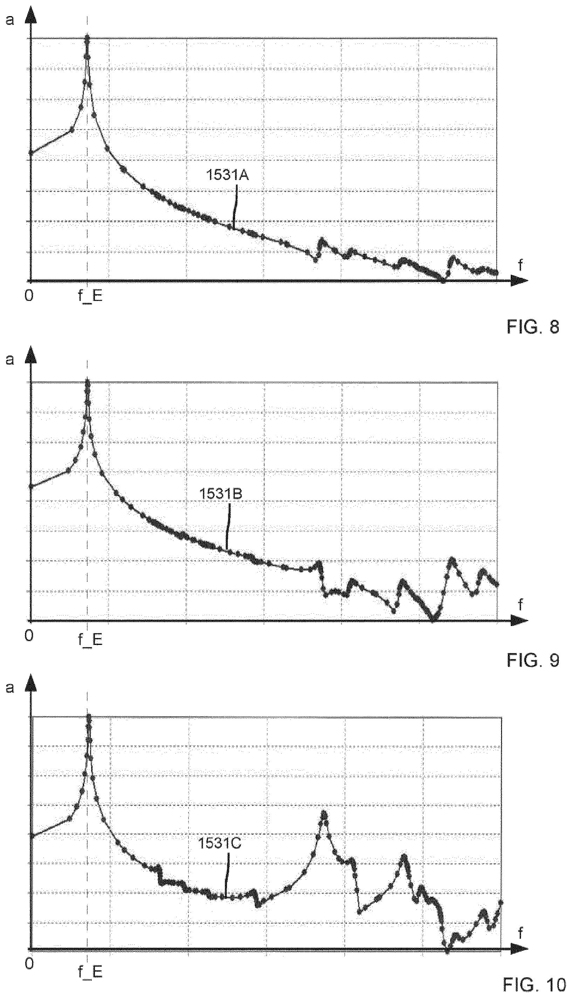

Läuft die mindestens eine Rolle 161 mit einem Abstand A zu einer optionalen nächsten Rolle 161 entlang des Rollwegs 145 in der Transportrichtung TR, werden mit dem Sensor 151 nacheinander verschiedene Erfassungsergebnisse 1531A, 1531B,1531C erfasst, die in Bezug auf Fig. 8 bis Fig. 10 noch genauer erläutert sind.

Zur Auswertung des Zustands, insbesondere der Laufeigenschaften, einer einzelnen Rolle 161 ist der Rollweg 145 in seiner mechanischen Struktur derart ausgestaltet, dass der Sensor 151 in der Aussparung 146 sehr empfindlich auf Körperschall im Bereich der Erfassungsstrecke 144 ist. Dagegen kann der Sensor 151 in der Aussparung 146 Körperschall außerhalb der Erfassungsstrecke 144 kaum erfassen, da Anregungen außerhalb der Erfassungsstrecke 144 stark unterdrückt bzw. gedämpft werden.

Die schwingungsmäßige Entkopplung des zweiten Führungskurvenbereichs 142, einschließlich des darin in der Aussparung 146 angeordneten Sensors 151, von dem ersten Führungskurvenbereich 141 erfolgt durch eine gezielte Auslegung der Masse des zweiten Führungskurvenbereichs 142 und die Steifigkeit der Anbindung des ersten und zweiten Führungskurvenbereichs 141, 142 aneinander.

Wie in Zusammenschau von Fig. 4 und Fig. 5 ersichtlich, ist die Anbindung 148 des zweiten Führungskurvenbereichs 142 derart gewählt, dass sich eine geringe Steifigkeit in einer vorbestimmten Erfassungsrichtung MR ergibt, in welcher die Schwingungen erfasst werden. In den beiden anderen Richtungen des dreidimensionalen Raums ist die Anbindung 148 des zweiten Führungskurvenbereichs 142 steifer als in der Erfassungsrichtung MR ausgelegt. Vergleichsweise ist die Anbindung 148 des zweiten Führungskurvenbereichs 142 in den beiden anderen Richtungen des dreidimensionalen Raums sehr steif. Je nach Anwendung kann zusätzlich das Material der Erfassungsstrecke 144 weicher sein bzw. eine geringere Steifigkeit haben als das Material des übrigen Rollwegs 145 außerhalb der Erfassungsstrecke 144. Die Ausgestaltung der Anbindung 148 und die Aussparung 143 bewirken, dass die Erfassungsstrecke 144 und der Rollweg 145 schwingungsmäßig entkoppelt voneinander sind.

Gemäß Fig. 4 und Fig. 5 werden die Beschleunigungswerte senkrecht zum Rollweg 145 erfasst, der zum Abrollen der Lauffläche der Rolle 161 bzw. zum Abwälzen des Außenumfangs des Wälzkörpers 1611 dient. Der Rollweg 145 liegt in einer Ebene, die in etwa senkrecht zu der vorbestimmten Erfassungsrichtung MR angeordnet ist.

Die Kombination aus Masse und Steifigkeit des zweiten Führungskurvenbereichs 142 einschließlich des Sensors 151 ergibt eine Eigenfrequenz, mit welcher der zweite Führungskurvenbereich 142 in Erfassungsrichtung MR schwingt, wie in Fig. 5 veranschaulicht.

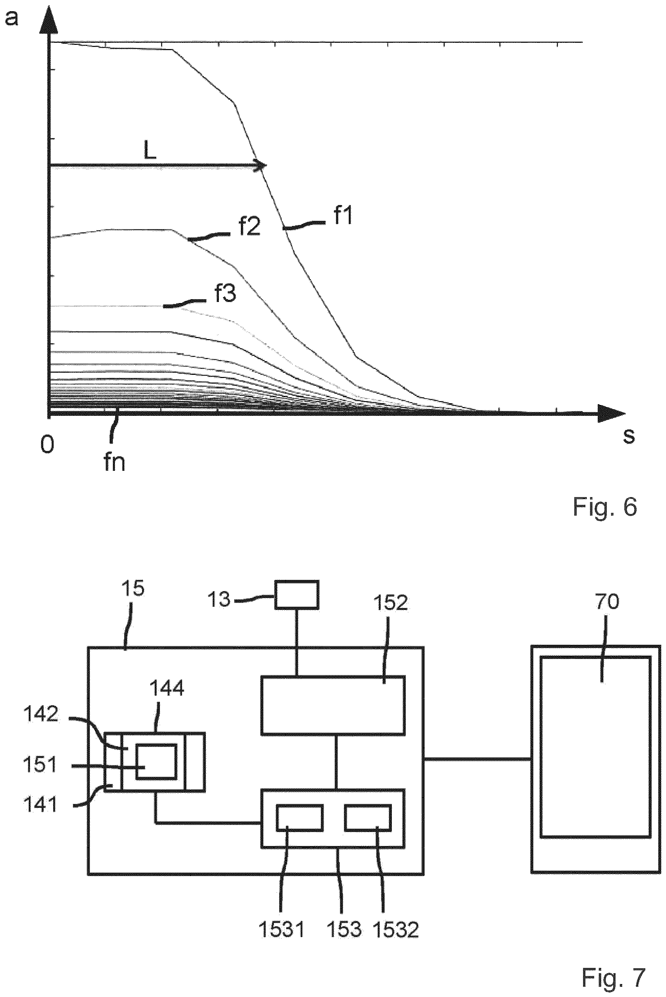

Wie in Fig. 6 für verschiedene Schwingungsfrequenzen f1, f2, f3, ... fn über dem Weg s ausgehend bei 0 als der Symmetrielinie 147 dargestellt, werden aufgrund dieser Anordnung Bewegungen a, wie Körperschall bzw. dadurch verursachte Schwingungen mit den Frequenzen f1, f2, f3, ... fn, sehr stark unterdrückt, die sowohl ihren Ursprung an einem Weg s außerhalb der Erfassungsstrecke 144 (Lange L) haben als auch eine größere Frequenz f als die Eigenfrequenz des Führungskurvenbereichs 142 einschließlich des Sensors 151 haben und in Richtung der Erfassungsrichtung MR weisen.

Andererseits werden Bewegungen a, wie Körperschall bzw. dadurch verursachte Schwingungen mit den Frequenzen f1, f2,f3, ... fn, die ihren Ursprung in der Länge L der Erfassungsstrecke 144 haben und in Erfassungsrichtung MR weisen, eine hohe Amplitude der Beschleunigung a zur Folge haben, wie in Fig. 6 gezeigt.

Die Erfassungsstrecke 144 ist zwischen der Aussparung 143 derart lang ausgelegt, dass die Länge L der Erfassungsstrecke 144 minimal genau einer Abwicklung einer Umdrehung des Wälzkörpers 1611 einer Rolle 161 entspricht. Die Länge L der Erfassungsstrecke 144 entspricht somit mindestens dem Außenumfang des Wälzkörpers 1611 einer Rolle 161. Die maximale Länge L der Erfassungsstrecke 144 hängt mit dem Abstand A der Rollen 161 zusammen, was nachfolgend noch genauer erläutert ist.

Als Folge davon werden mit der Erfassungseinrichtung 15 als die Erfassungsergebnisse 1531A bis 1531C Beschleunigungsamplituden erfasst, die eine höhere Frequenz als die Eigenfrequenz des zweiten Führungskurvenbereichs 142 einschließlich des Sensors 151 aufweisen.

Gemäß Fig. 7 hat die Erfassungseinrichtung 15 zusätzlich zu dem Sensor 151 ein Auswertemodul 152 und ein Speichermodul 153. Der Sensor 151 ist insbesondere als Beschleunigungssensor ausgestaltet. Die Erfassungsergebnisse 1531A bis 1531C des Sensors 151 werden als Istwert oder Erfassungsergebnis 1531 in dem Speichermodul 153 gespeichert. Zudem ist in dem Speichermodul 153 mindestens ein Sollwert 1532 gespeichert, der einem Erfassungsergebnis einer idealen Rolle 161 entspricht. Der Sollwert 1532 ist insbesondere eine Referenzkurve. Das Erfassungsergebnis 1531 bzw. die Erfassungsergebnisse 1531A bis 1531C und der Sollwert 1532 sind jeweils einer Ausgabe des Drehgebers 13 zugeordnet. Somit kann das Auswertemodul 152 das Erfassungsergebnis 1531 bzw. die Erfassungsergebnisse 1531A bis 1531C und den Sollwert 1532 lagerichtig an der Führungskurve 14 zueinander auswerten. Damit können die Erfassungsergebnisse 1531A bis 1531C den einzelnen Rollen 161 an der Führungskurve 14 zugeordnet werden.

Das Auswerteergebnis und/oder das Erfassungsergebnis 1531 bzw. die Erfassungsergebnisse 1531A bis 1531C und der Sollwert 1532 sind an eine Bedieneinrichtung 70 übertragbar. Dadurch ist es möglich, dass ein Bediener der Behälterbehandlungsanlage 1 auf Anforderung oder automatisch über die Laufeigenschaften mindestens einer der Rollen 161 informiert wird. Aus den Laufeigenschaften ist durch Vergleich mit dem Sollwert 1532 der Verschleißzustand der jeweiligen Rolle 161 ableitbar. Beispielsweise kann für eine noch tolerierbaren Abnutzung oder Verschleiß ein vorbestimmtes Toleranzband definiert sein. Liegen die Laufeigenschaften der betrachteten Rolle 161 außerhalb des Toleranzbands wird die Rolle 161 als verschlissen beurteilt. Ansonsten kann die betrachtete Rolle 161 weiterverwendet werden. Anstelle eines Toleranzbandes kann jedoch auch nur eine Grenze festgelegt sein, bei deren Überschreiten die Rolle 161 als verschlissen beurteilt wird.

Fig. 8 bis Fig. 10 zeigen Beispiele für die Ausgaben der Erfassungsergebnisse 1531A bis 1531C. Hierbei ist jeweils nur die Schwingung bzw. Beschleunigung a in Bezug auf eine Frequenz f dargestellt. Das Erfassungsergebnis 1531A gemäß Fig. 8 entspricht einer Erfassung der Schwingungen bzw. deren Frequenzen f an der Erfassungsstelle in Messrichtung MR des Sensors 151, wenn durch eine Rolle 161 eine Schwingung mit der Beschleunigung a mit immer der gleichen Amplitude jedoch veränderlicher Frequenz f bei einer Position eingekoppelt wird/werden, die um einen Winkel von ca. 4° gegenüber der Symmetrielinie 147 versetzt angeordnet ist, wie in gezeigt.

Das Erfassungsergebnis 1531B gemäß Fig. 4 Fig. 9 entspricht einer Erfassung der Schwingungen bzw. deren Frequenzen f für eine Rolle 161, die anstelle der Position von 4° gemäß Fig. 8 bei einer Position angeordnet ist, die um einen Winkel von ca. 16° gegenüber der Symmetrielinie 147 versetzt angeordnet ist, wie in gezeigt. Das Erfassungsergebnis 1531C gemäß Fig. 4 Fig. 10 entspricht einer Erfassung der Schwingungen bzw. deren Frequenzen f für eine Rolle 161, die anstelle der Position von 4° gemäß Fig. 8 bei einer Position eingekoppelt wird/werden, die um einen Winkel von ca. 28° gegenüber der Symmetrielinie 147 versetzt angeordnet ist, wie in Fig. 4 gezeigt.

Fig. 8 bis Fig. 10 geben somit jeweils ein Frequenzspektrum für die verschiedenen Positionen der Rollen 161 an der Führungskurve 14 an. Daraus lässt sich deutlich die Eigenfrequenz f_E der mechanischen Struktur des zweiten Führungskurvenbereichs 142 ablesen, nämlich als die Spitze der erfassbaren Amplituden der Beschleunigung a über der Frequenz f bei den jeweiligen Positionen 4°, 16°, 28°.

Hierbei sind in Fig. 8 bis Fig. 10 die vertikalen Skalierungen der Darstellung unterschiedlich gewählt. Effektiv sind die Amplituden des Erfassungsergebnisses 1531A für die Beschleunigung a am größten und die Amplituden des Erfassungsergebnisses 1531C für die Beschleunigung a am kleinsten, auch wenn sich dies aus dem direkten Vergleich von Fig. 8 bis Fig. 10 nicht ergibt. Dies stimmt mit der Darstellung von Fig. 6 überein, gemäß welcher die Amplituden der erfassten Beschleunigung a mit zunehmender Entfernung von der Symmetrielinie 147 abnehmen.

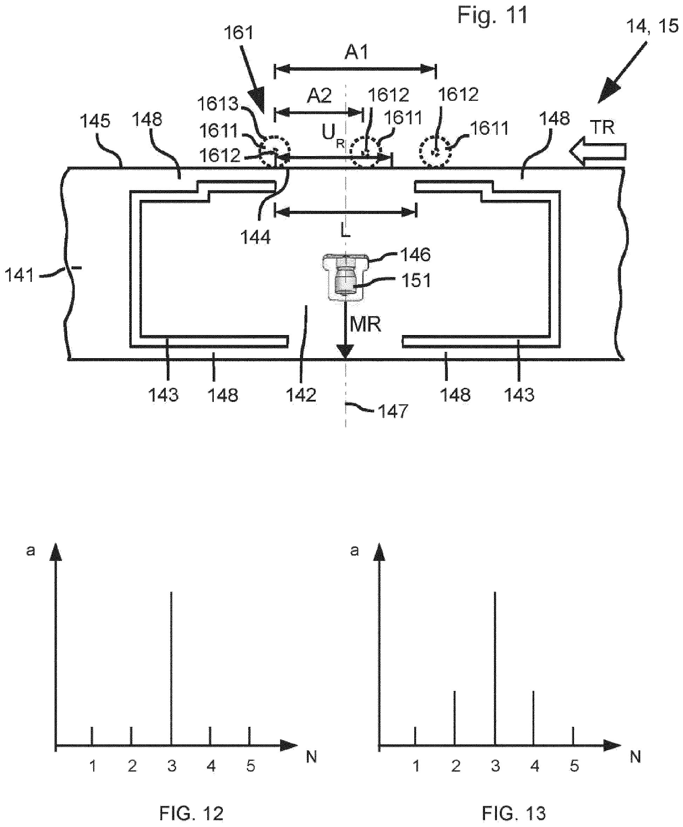

Fig. 11 veranschaulicht zwei Fälle, nämlich einen ersten Fall, bei welchem zwischen den Achsen 1612 von zwei aufeinanderfolgenden Rollen 161 ein Abstand A1 vorhanden ist, und einen zweiten Fall, bei welchem zwischen den Achsen 1612 von zwei aufeinanderfolgenden Rollen 161 ein Abstand A2 vorhanden ist. Hierbei ist die Abwicklung U einer R Umdrehung des Wälzkörpers 1611 bzw. der Außenumfang 1613 einer Rolle 161 kleiner als die Länge L der Erfassungsstrecke 144.

Das Auswertemodul 152 der Erfassungseinrichtung 15 berechnet für ein einer Rolle 161 zugeordnetes Auswerte-Winkelintervall der Maschine 10 die Amplitude der Beschleunigung a oder ein Erfassungssignal als Schwingungseffektivwert, wobei pro Rolle 161 eine Amplitude der Beschleunigung a berechnet wird. Pro Umdrehung der Maschine 10 bzw. pro Umlauf einer Rolle 161 um die Maschine 10 wird diese Amplitude der Beschleunigung a neu berechnet und über eine Mittelung, die insbesondere mit einem Mittelungsalgorithums des Auswertemoduls 152 ausführbar ist, mit vorausgehenden Erfassungswerten verrechnet.

Das Ergebnis dieser Auswertung ist in Fig. 12 für fünf aufeinanderfolgende Rollen 161 für den ersten Fall gezeigt, bei dem der Abstand A1 zwischen den Achsen 1612 von zwei aufeinanderfolgenden Rollen 161 vorhanden ist. In Fig. 13 ist das Ergebnis dieser Auswertung für fünf aufeinanderfolgende Rollen 161 für den zweiten Fall gezeigt, bei dem der Abstand A2 zwischen den Achsen 1612 von zwei aufeinanderfolgenden Rollen 161 vorhanden ist.

Wie aus Fig. 12 erkennbar, ist bei dem ersten Fall von Fig. 11 eine sehr scharfe Abgrenzung der Amplitude der Beschleunigung a der dritten Rolle 161 auf der Erfassungsstrecke 144 von der Amplitude der Beschleunigung a der benachbarten Rollen 161 erkennbar, die nicht auf der Erfassungsstrecke 144 angeordnet sind. In diesem Fall werden im jeweiligen Auswerte-Winkelintervall für die dritte Rolle 161 auf der Erfassungsstrecke 144 annähernd ausschließlich Schwingungen gemessen, die von dieser Rolle 161 selbst stammen.

Wie aus Fig. 13 erkennbar, ist bei dem zweiten Fall von Fig. 11 eine weniger scharfe Abgrenzung der Amplitude der Beschleunigung a der dritten Rolle 161 auf der Erfassungsstrecke 144 von der Amplitude der Beschleunigung a der benachbarten Rollen 161 erkennbar. Dies ergibt sich dadurch, dass auch die zweite und/oder vierte Rolle zumindest teilweise gleichzeitig mit der dritten Rolle über die Erfassungsstrecke 144 rollen.

Somit erhöht sich die Beschleunigungsamplitude von Rollen 161 ebenfalls, die benachbart zu einer verschlissenen Rolle 161 angeordnet sind. Der Grund dafür liegt darin, dass sich die Auswerte-Winkelintervalle zur Berechnung einer Beschleunigungsamplitude überlappen. Somit ist bei dem in Fig. 13 veranschaulichten Fall die Amplitude der Beschleunigung a für die zweite und vierte Rolle 161 ebenfalls erhöht, wenn die dritte Rolle 161 verschlissen ist. Die Erhöhung des Schwingungseffektivwerts ist aber trotzdem bei der jeweils auszuwertenden Rolle 161 am höchsten.

Somit ist sowohl für den ersten Fall von Fig. 11 als auch für den zweiten Fall von Fig. 11 die selektive Auswertung der Laufeigenschaften der Rollen 161 an der Führungskurve 14 möglich. In anderen Worten, die Auswertung der Laufeigenschaften der Rollen 161 an der Führungskurve 14 ist mit der Erfassungseinrichtung 15 auch möglich, wenn die Länge L der Erfassungsstrecke 144 kleiner als der Abstand A () bzw. A1 () der Rollen 161 ist bzw. gilt L < A oder L < A1.

Somit werden mit der Erfassungseinrichtung 15 die Schwingungen erfasst und ausgewertet, die durch den abrollenden Wälzkörper 1611 einer einzelnen Rolle 161 in dem zweiten Führungskurvenbereich 142 in Erfassungsrichtung MR erzeugt werden. Hierbei werden die gemessenen Amplituden der Beschleunigung a durch Einbeziehung der Ausgabe des Drehgebers 13 einem bestimmten Maschinenwinkel der ersten Behälterbehandlungsmaschine 10 oder einer sonstigen Rundläufermaschine oder Rundläufertransporteinrichtung wie bei der Transporteinrichtung 50 oder der Transporteinrichtung 60 zugeordnet.

Auf diese Weise kann die selektive Auswertung der Laufeigenschaften mindestens einer Rolle 161 der Rollen 161 einfach und sicher durchgeführt werden, um den Verschleißzustand jeder einzelnen Rolle 161 an der Führungskurve 14 zu bestimmen.

Es ist auch möglich, das zuvor beschriebene Prinzip bei einer Anordnung anzuwenden, bei welcher die Rollen 161 nicht in einer Kreisbewegung über einen Rollweg 145 geführt werden sondern über einen Rollweg 145 hin und her bewegt werden können. Auch hier kann eine Erfassungsstrecke 144 vorhanden sein, welche die Laufeigenschaften einer einzelnen Rolle 161 erfassen kann. Hier ist es gegebenenfalls denkbar, von allen Rollen 161, die auf dem Rollweg 145 laufen, nur die Laufeigenschaften mindestens einer einzelnen Rolle 161 mit einer Erfassungseinrichtung 15 zu erfassen. Von den Laufeigenschaften der mindestens einen einzelnen Rolle 161 kann dann auf die Laufeigenschaften der anderen Rollen 161 zurückgeschlossen werden. Auch wenn dies in Bezug auf die Erfassung der Laufeigenschaften für jede einzelne Rolle 161 weniger optimal ist, so kann eine derartige Anordnung dennoch bei Bedarf ausreichend sein und bietet in jedem Fall eine Verbesserung gegenüber dem Stand der Technik.

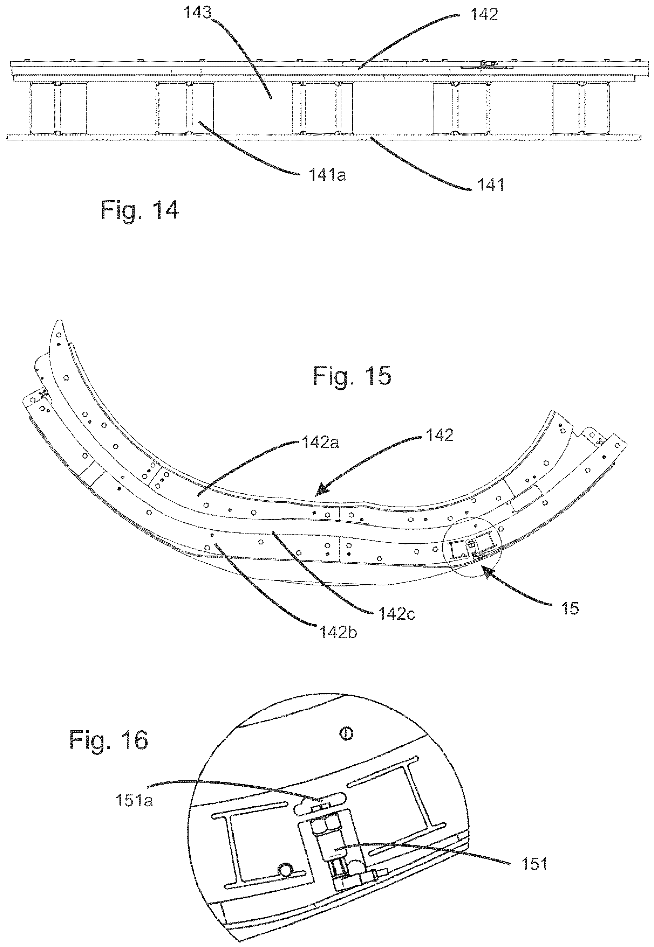

Fig. 14 zeigt eine weitere Darstellung einer Führungskurve. Diese Führungskurve ist insbesondere für den Betrieb einer Umformungseinrichtung zum Umformen von Kunststoffvorformlingen zu Kunststoffbehältnissen bestimmt.

Die Führungskurve weist einen ersten Führungskurventeil 141 und einen zweiten Führungskurventeil 142 auf. Diese Führungskurventeile sind durch einen Spalt 143 voneinander beabstandet und mittels Verbindungseinrichtungen 141a miteinander verbunden.

Dabei ist eine Vielzahl solcher Verbindungseinrichtungen 141a bevorzugt in regelmäßigen Abständen zueinander vorgesehen.

Fig. 15 zeigt eine Draufsicht auf die in Fig. 14 gezeigte Führungskurve, genauer auf den zweiten Führungskurventeil 142.

Man erkenn, dass dieser zweite Führungskurventeil 142 einen ersten Führungssteg 142a und einen zweiten Führungssteg 142b aufweist, zwischen denen ein Spalt 142c mit bevorzugt einheitlicher Breite angeordnet ist. Innerhalb dieses Stegs können (nicht gezeigte) Kurvenrollen abrollen und zwar bevorzugt sowohl gegenüber dem ersten Führungssteg 142a als auch gegenüber dem zweiten Führungssteg 142b.

Das Bezugszeichen 15 kennzeichnet die Erfassungseinrichtung.

Fig. 16 zeigt eine vergrößerte Darstellung der Erfassungseinrichtung. Auch hier ist wieder der Sensor 151 dargestellt.

Alle zuvor beschriebenen Ausgestaltungen der Behälterbehandlungsanlage 1 und des von ihr ausgeführten Verfahren zur Erfassung von Laufeigenschaften einer Rolle 161 an einer Führungskurve 14, 24, 34, 54, 64 können einzeln oder in allen möglichen Kombinationen Verwendung finden. Insbesondere können die Merkmale der zuvor beschriebenen Ausführungsbeispiele beliebig kombiniert oder auch weggelassen werden. Zusätzlich sind insbesondere folgende Modifikationen denkbar.

Die in den Figuren dargestellten Teile sind schematisch dargestellt und können in der genauen Ausgestaltung von den in den Figuren gezeigten Formen abweichen, solange deren zuvor beschriebenen Funktionen gewährleistet sind.

Mindestens eine der Behälterbehandlungsmaschinen 10, 20, 30, 40 kann eine Streckblasmaschine zum Herstellen von Behältern 2 aus Preforms sein. Mindestens eine der Behälterbehandlungsmaschinen 10, 20, 30, 40 kann eine Heizeinrichtung sein, die in der Streckblasmaschine zum Heizen der Preforms verwendet wird. Mindestens eine der Behälterbehandlungsmaschinen 10, 20 , 30, 40 kann eine Inspektionsmaschine zum Inspizieren der Behälter 2 auf Fehler sein. Mindestens eine der Behälterbehandlungsmaschinen 10, 20, 30, 40 kann eine Reinigungsmaschine zum Reinigen des mindestens einen Behälters 2 sein.

Es sind selbstverständlich auch alle anderen Behälterbehandlungsmaschinen als Behälterbehandlungsmaschine(n) 10, 20, 30 möglich, die zur Behandlung von Behältern 2 ausgestaltet sind. Die Behälterbehandlungsanlage 1 kann in beliebiger Anzahl, Kombination und Ausführung Behälterbehandlungsmaschinen 10, 20, 30, 40, und/oder weitere Behälterbehandlungsmaschinen, wie zuvor beschrieben, gegebenenfalls davor und/oder dazwischen und/oder danach angeordneten Transporteinrichtungen 50, 60 in beliebiger Anzahl, Kombination und Ausführung aufweisen.

Die Anmelderin behält sich vor sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

## Patentansprüche

1. Vorrichtung (10, 20, 30, 40) zum Behandeln von Behältnissen (2) mit einer Transporteinrichtung (50), welche die zu behandelnden Behältnisse (2) entlang eines vorgegebenen Transportpfads (TR) transportiert und mit wenigstens einer Behandlungseinrichtung (17, 18, 26, 27, 36, 37, 38, 39, 162), welche dazu geeignet und bestimmt ist, ein Behältnis (2) in einer vorgegebenen Weise zu behandeln, wobei die Vorrichtung (1) eine Bewegungseinrichtung (14, 24, 34, 54, 64, 161) aufweist, und diese Bewegungseinrichtung wenigstens eine erste Führungskurve (14, 24, 34, 54, 64) aufweist, sowie wenigstens eine Führungsrolle (161), welche dazu geeignet und bestimmt ist, gegenüber der Führungskurve (14, 24, 34, 54, 64) abzurollen und wobei die Vorrichtung (1) weiterhin eine Erfassungseinrichtung (15, 65) aufweist, welche dazu geeignet und bestimmt ist, wenigstens einen Messwert zu erfassen, der für eine Laufeigenschaft der Führungsrolle (161) gegenüber der Führungskurve (14, 24, 34, 54, 64) charakteristisch ist,
**dadurch gekennzeichnet, dass**
die Vorrichtung (1) eine Auswerteeinrichtung (100) aufweist, welche dazu geeignet und bestimmt ist, diesen erfassten Messwert unter Berücksichtigung wenigstens eines weiteren für den Betrieb der Vorrichtung charakteristischen Betriebswertes auszuwerten.

2. Vorrichtung (10, 20, 30, 40) nach Anspruch 1,
**dadurch gekennzeichnet, dass**
die Vorrichtung (1) eine Zuordnungseinrichtung (102) aufweist, welche dazu geeignet und bestimmt ist, dem Messwert einen für den Betrieb der Vorrichtung charakteristischen Betriebswert zuzuordnen und insbesondere einen Betriebswert, der für einen Zeitraum oder Zeitpunkt charakteristisch ist, in oder zu welchem der Messwert aufgenommen wurde.

3. Vorrichtung (10, 20, 30, 40) nach wenigstens einem der vorangegangenen Ansprüche,
**dadurch gekennzeichnet, dass**
die Vorrichtung (1) eine Speicherungseinrichtung (104) aufweist, welche zum Speichern einer Vielzahl von Messwerten geeignet und bestimmt ist, wobei bevorzugt diese Messwerte mit einer Zuordnung zu diesen Messwerten zugeordneten Betriebswerten abspeicherbar sind.

4. Vorrichtung (10, 20, 30, 40) nach wenigstens einem der vorangegangenen Ansprüche,
**dadurch gekennzeichnet, dass**
die Vorrichtung (1) eine Vielzahl von Behandlungseinrichtungen aufweist, welche dazu geeignet und bestimmt sind, Behältnisse (2) in einer vorgegebenen Weise zu behandeln, wobei jeder dieser Behandlungseinrichtungen wenigstens eine Kurvenrolle zugeordnet ist.

5. Vorrichtung (1) nach wenigstens einem der vorangegangenen Ansprüche,
**dadurch gekennzeichnet, dass**
die Vorrichtung (1) eine Vergleichseinrichtung (106) aufweist, welche gemessene Messwerte mit wenigstens einem Sollwert vergleicht und bevorzugt wenigstens einen für diesen Vergleich charakteristischen Vergleichswert ausgibt.

6. Vorrichtung (1) nach dem vorangegangenen Anspruch,
**dadurch gekennzeichnet, dass**
die Vorrichtung (1) eine Auswahleinrichtung aufweist, welche dazu geeignet und bestimmt ist, einen Sollwert aus einer Vielzahl von Sollwerten auszuwerten.

7. Vorrichtung (1) nach wenigstens einem der vorangegangenen Ansprüche,
**dadurch gekennzeichnet, dass**
die Vorrichtung wenigstens eine weitere Sensoreinrichtung (...) aufweist, welche dazu geeignet und bestimmt ist, wenigstens einen für einen für Einflussfaktoren charakteristischen Wert aufzunehmen.

8. Vorrichtung nach einem der vorangehenden Ansprüche,
**dadurch gekennzeichnet, dass**
die Erfassungseinrichtung (15) zudem ein Auswertemodul (152) aufweist, das dazu geeignet und bestimmt ist, Messwerte den einzelnen Rollen (161) an der Führungskurve (14; 24; 34; 54; 64) zuzuordnen.

9. Vorrichtung nach dem vorangegangenen Anspruch,
**dadurch gekennzeichnet, dass**
die Zuordnung unter Berücksichtigung vorgegebener Daten durchführbar ist, wobei bevorzugt die Daten aus einer Gruppe von Daten ausgewählt sind, welche einen Drehwinkel insbesondere eines Transportträgers oder eine Position einer Transporteinrichtung, die Position einer Behandlungseinrichtung und/oder eine Zeitinformation, oder ein Signal eines Drehgebers enthält.

10. Vorrichtung nach einem der vorangehenden Ansprüche,
**dadurch gekennzeichnet, dass**
die Vorrichtung (1) aus einer Gruppe von Vorrichtungen ausgewählt ist, welche Umformungseinrichtungen zum Umformen von Kunststoffvorformlingen zu Kunststoffbehältnissen, Fülleinrichtungen zum Befüllen von Behältnissen, Sterilisiereinrichtungen zum Sterilisieren von Behältnissen, Erwärmungseinrichtungen zum Erwärmen von Kunststoffvorformlingen, Druckeinrichtungen zum Bedrucken von Behältnissen, Etikettiereinrichtungen zum Etikettieren von Behältnissen, Verschließeinrichtungen zum Verschließen von Behältnissen mit Behältnisverschlüssen, Packeinrichtungen zum Verpacken von Behältnissen und Reinigungseinrichtungen zum Reinigen von Behältnissen enthält.

11. Vorrichtung nach einem der vorangehenden Ansprüche,
**dadurch gekennzeichnet, dass**
die Erfassungseinrichtung wenigstens eine Sensoreinrichtung aufweist, welche wenigstens einen Messwert erfasst, der für wenigstens eine Bewegungsgröße der Führungsrolle oder der Behandlungseinrichtung oder der Halteeinrichtung charakteristisch ist, wobei die Sensoreinrichtung bevorzugt aus einer Gruppe von Sensoreinrichtungen ausgewählt ist, welche Schwingungssensoren, Geschwindigkeitssensoren, Kraftsensoren, Beschleunigungssensoren, Abstandssensoren, Akustik- oder Schallsensoren und dergleichen enthält.

12. Behältnisbehandlungsanlage (50) mit wenigstens einer Vorrichtung (1) nach wenigstens einen der vorangegangenen Ansprüche,
**dadurch gekennzeichnet, dass**
die Behältnisbehandlungsanlage eine weitere Vorrichtung zum Behandeln von Behältnissen aufweist und eine Erfassungseinrichtung vorgesehen ist, welche dazu geeignet und bestimmt wenigstens einen für diese weitere Vorrichtung charakteristischen weiteren Messwert zu erfassen, wobei bevorzugt die Auswerteeinrichtung dazu geeignet und bestimmt ist, auch diesen weiteren Messwert bei der Auswertung zu berücksichtigen.

13. Verfahren zum Behandeln von Behältnissen (10, 15) wobei eine Transporteinrichtung (2) die zu behandelnden Behältnisse (10, 20) entlang eines vorgegebenen Transportpfads transportiert und wenigstens eine Behandlungseinrichtung wenigstens ein Behältnis in einer vorgegebenen Weise behandelt, wobei die Vorrichtung (1) eine Bewegungseinrichtung aufweist, und diese Bewegungseinrichtung wenigstens eine erste Führungskurve aufweist, sowie wenigstens eine Führungsrolle, welche gegenüber der Führungskurve (..) abrollt und wobei die Vorrichtung (1) weiterhin eine Erfassungseinrichtung (...) aufweist, welche wenigstens einen Messwert erfasst, der für eine Laufeigenschaft der Führungsrolle gegenüber der Führungskurve charakteristisch ist,
**dadurch gekennzeichnet, dass**
die Vorrichtung (1) eine Auswerteeinrichtung (100) aufweist, welche diesen erfassten Messwert unter Berücksichtigung wenigstens eines weiteren für den Betrieb der Vorrichtung charakteristischen Wertes auswertet.

14. Verfahren nach Anspruch 12,
**dadurch gekennzeichnet, dass**
wenigstens ein weiterer für den Behandlungsvorgang charakteristischer Parameter erfasst wird und bevorzugt dieser weitere Parameter bei der Auswertung berücksichtigt wird.

15. Verfahren nach dem vorangegangenen Anspruch,
**dadurch gekennzeichnet, dass**
der weitere charakteristische Parameter bei der Ermittlung von Sollwerten berücksichtigt wird.
